(19) Europäisches Patentamt
European Patent Office
Office européen des brevets

(11) EP 2 764 126 B1

(12) **FASCICULE DE BREVET EUROPEEN**

(45) Date de publication et mention de la délivrance du brevet:
**29.11.2017 Bulletin 2017/48**

(21) Numéro de dépôt: **12779108.5**

(22) Date de dépôt: **03.10.2012**

(51) Int Cl.:
***C12R 1/125*** *(2006.01)* ***C12P 7/64*** *(2006.01)*
***C12P 21/00*** *(2006.01)*

(86) Numéro de dépôt international:
**PCT/FR2012/052234**

(87) Numéro de publication internationale:
**WO 2013/050700 (11.04.2013 Gazette 2013/15)**

(54) **BIOSURFACTANTS PRODUITE PAR UNE NOUVELLE SOUCHE DE BACILLUS SUBTILIS, COMPOSITION LES COMPRENANT, PROCEDE D'OBTENTION ET APPLICATION**

VON EINEM NEUEN BACILLUS SUBTILIS-STAMM HERGESTELLTE BIOLOGISCHE TENSIDZUSAMMENSETZUNG, ZUSAMMENSETZUNGEN, DIE DIESE ENTHALTEN UND ANWENDUNG

BIOSURFACTANTS PRODUCED BY A NOVEL STRAIN OF BACILLUS SUBITILIS, COMPOSITIONS CONTAINING THEM, PROCESS FOR OBTAINING AND USE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **03.10.2011 FR 1158922**

(43) Date de publication de la demande:
**13.08.2014 Bulletin 2014/33**

(73) Titulaire: **Université de Lille 1 Sciences et Technologies**
**59655 Villeneuve d'Ascq Cedex (FR)**

(72) Inventeurs:
- **COUTTE, François**
  **F-59000 Lille (FR)**
- **JACQUES, Philippe**
  **B-4000 Liège (BE)**
- **LECOUTURIER, Didier**
  **F-59152 Chéreng (FR)**
- **GUEZ, Jean-Sébastien**
  **F-63960 Veyre-Monton (FR)**
- **DHULSTER, Pascal**
  **F-59000 Lille (FR)**
- **LECLERE, Valérie**
  **F-59650 Villeneuve-d'Ascq (FR)**
- **BECHET, Max**
  **F-59650 Villeneuve-d'Ascq (FR)**

(74) Mandataire: **IP Trust**
**2, rue de Clichy**
**75009 Paris (FR)**

(56) Documents cités:
- **PATRICK FICKERS ET AL: "High-Level Biosynthesis of the Anteiso-C17 Isoform of the Antibiotic Mycosubtilin in Bacillus subtilis and Characterization of Its Candidacical Activity", APPLIED AND ENVIRONMENTAL MICROBIOLOGY, vol. 75, no. 13, juillet 2009 (2009-07), pages 4636-4640, XP002707276,**
- **V. LECLERE ET AL: "Mycosubtilin Overproduction by Bacillus subtilis BBG100 Enhances the Organism's Antagonistic and Biocontrol Activities", APPLIED AND ENVIRONMENTAL MICROBIOLOGY, vol. 71, no. 8, 1 août 2005 (2005-08-01), pages 4577-4584, XP055046643, ISSN: 0099-2240, DOI: 10.1128/AEM.71.8.4577-4584.2005 cité dans la demande**
- **F. COUTTE ET AL: "Production of surfactin and fengycin by Bacillus subtilis in a bubbleless membrane bioreactor", APPLIED MICROBIOLOGY AND BIOTECHNOLOGY, SPRINGER, BERLIN, DE, vol. 87, no. 2, 11 mars 2010 (2010-03-11), pages 499-507, XP019841523, ISSN: 1432-0614 cité dans la demande**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- DUITMAN ET AL: "The mycosubtilin synthetase of Bacillus subtilis ATCC6633: A multifunctional hybrid between a peptide synthetase, an amino transferase, and a fatty acid synthase", PROCEEDINGS OF THE NATIONAL ACADEMY OF SCIENCES, NATIONAL ACADEMY OF SCIENCES, US, vol. 96, no. 23, 9 novembre 1999 (1999-11-09), pages 13294-13299, XP002145095, ISSN: 0027-8424, DOI: 10.1073/PNAS.96.23.13294 cité dans la demande
- COUTTE, F. ET AL.: "Effect of pps disruption and constitutive expression of srfA on surfactin productivity, spreading and antagonistic properties of Bacillus subtilis 168 derivatives", J. APPL. MICROBIOL., vol. 109, 2010, pages 480-491, XP2632917, cité dans la demande
- T.STEIN: "Whole-cell matrix assisted laser desorption/ionization matrix spectroscopy for rapid identification of bacteriocin/lantibiotic-producing bacteria", RAPID COMMUNICATIONS IN MASS SPECTROSCOPY, vol. 22, no. 8, 30 April 2008 (2008-04-30) , pages 1146-1152, DOI: 10.1002/rcm.3481

## Description

### Domaine technique

La présente invention se rapporte à des biosurfactants produits par une souche de *Bacillus sp.* et à leurs utilisations. Elle se rapporte également à une composition comprenant ces biosurfactants,

La présente invention trouve notamment des applications dans la production de biopesticides ou biosurfactants pour l'industrie phytosanitaire, mais également dans les domaines des industries alimentaire, cosmétique, chimique, pharmaceutique, pétrolière et de l'environnement.

Dans la description ci-dessous, les références entre crochets (**[ ]**) renvoient à la liste des références présentée à la fin des exemples.

### État de la technique

Le système de production agricole conventionnel utilise des produits phytosanitaires de type pesticides pour assurer une production suffisante en quantité et en qualité, conforme aux attentes des marchés et à un coût acceptable pour le consommateur. Si l'utilisation de ces produits apporte des bénéfices pour les systèmes agricoles, elle peut néanmoins être à l'origine d'effets négatifs pour la santé humaine et pour l'environnement. La dégradation de la qualité des eaux souterraines et des eaux de surface et la réduction de la biodiversité en milieu agricole sont les conséquences les plus fréquemment citées.

Les biosurfactants, notamment d'origine bactérienne, sont connus pour avoir de nombreuses propriétés intéressantes, en particulier des propriétés tensio-actives, antivirales, antibactériennes et antifongiques susceptibles d'être exploitées dans le domaine phytosanitaire. Ces biosurfactants peuvent être utilisés seuls ou dans un mélange de plusieurs biosurfactants. Des effets synergiques ont été montrés lorsque les biosurfactants sont utilisés sous forme de mélanges (Ongena and Jacques, 2008. Bacillus lipopeptides: versatile weapons for plant disease biocontrol, Trends Microbiol. 16, 115-125 **[1]** ; CZ20011620 **[2]** ; DE102005050123 **[3]**).

Ces biosurfactants présentent une meilleure biodégradabilité, une toxicité plus basse et une résistance physico-chimique plus importante, comparées à un pesticide d'origine chimique. Par ailleurs, le marché cosmétique est particulièrement intéressé par des molécules d'origine biologique qui combinent des activités antimicrobiennes et des propriétés physico-chimiques comme des émulsifiants.

Les biosurfactants sont également utilisés dans la récupération assistée du pétrole contenu dans les gisements où l'injection de biosurfactants permet de réduire la viscosité du pétrole et d'améliorer sensiblement la proportion de pétrole récupérée. Ils sont également utilisés pour lutter contre les pollutions de l'eau par des hydrocarbures et sont beaucoup plus efficaces que les tensio-actifs chimiques. En outre, ces biosurfactants ne sont pas toxiques pour l'écosystème des eaux traitées.

Les demandes en biosurfactants se sont donc faites de plus en plus fortes au cours des dernières années, notamment dans les industries alimentaire, cosmétique, chimique, pharmaceutique, pétrolière et de l'environnement. De nombreux procédés de production ont été étudiés, mis en oeuvre et ont fait l'objet de publications ou de dépôts de demande de brevet (FR2578552 **[4]**).

Toutefois, les biosurfactants actuellement disponibles sont peu efficaces et présentent des propriétés biologiques et/ou chimiques limitées.

Il existe donc un réel besoin de fournir des biosurfactants alternatifs, de préférence ayant des propriétés améliorées par rapport aux biosurfactants de l'art antérieur.

Les procédés de production de biosurfactants produits par *Bacillus sp.* ont été particulièrement étudiés. Toutefois, ces procédés conduisent à la formation de mousse causée par l'apport d'oxygène, sous forme de bulles. Une première approche est d'utiliser des réacteurs aérés mécaniquement agités et d'extraire en continu la mousse provoquée par le biosurfactant et contenant ce dernier. Ce procédé est laborieux et peu exploitable, notamment à grande échelle (Guez et al., 2007. Setting up and modelling of overflowing fed-batch cultures of Bacillus subtilis for the production and continuous removal of lipopeptides. J. Biotechnol., 131, 67-75 **[5]**).

Afin d'éviter ce problème de mousse, il a été tenté de travailler en condition anaérobie et d'utiliser le nitrate comme accepteur final d'électrons (Davis, Lynch and Varley. 1999. The production of surfactin in batch culture by Bacillus subtilis ATCC 21332 is strongly influenced by the conditions of nitrogen metabolism. Enzyme Microb. Technol. 25, 322-329. **[6]** ; WO0226961**[7]** ; EP1320595 **[8]**). Les productions de biosurfactants dépendent fortement de la capacité des souches de s'adapter ou non à ces conditions anaérobies. Aucune solution efficace permettant de produire des biosurfactants en quantité industrielle n'a été mise au point à ce jour. Par ailleurs, l'utilisation de pesticides d'origine chimique étant de plus en plus contestée, il est nécessaire de rechercher et d'utiliser des molécules d'origine biologique pour remplacer les pesticides d'origine chimique et de mettre au point des procédés de production de ces molécules d'origine biologique à l'échelle industrielle.

Il existe donc de réels besoins de mettre au point un procédé palliant ces défauts, inconvénients et obstacles de l'art antérieur,
y compris un procédé permettant de produire en continu des biosurfactants, en quantités importantes, avec de faibles coûts de production.

**Description de l'invention**

La présente invention répond précisément aux besoins précités, en fournissant des mycosubtilines telles que revendiquées, et une composition comprenant ces mycosubtilines.

Ainsi la présente invention a pour objet la mycosubtiline suivante :

◦ Mycosubtiline Gln3 C17 : mycosubtiline dont la chaîne d'acide gras comporte 17 atomes de carbone et possédant une glutamine à la place de l'asparagine en position 3 dans son cycle peptidique et représentée par la formule (II) ci-dessous :

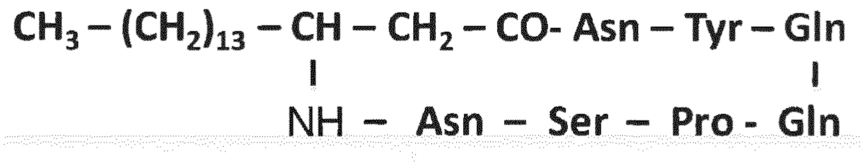

(II)

Les inventeurs ont également décrit une mycosubtiline C18, c'est-à-dire une mycosubtiline dont la chaîne d'acide gras comporte 18 atomes de carbone, et qui est représentée par la formule (I) ci-dessous :
(I)

Les mycosubtilines C18 et Gln3 C17 décrites ci-dessus peuvent être utilisées en tant qu'agent antifongique. Elles présentent d'ailleurs des effets antifongiques équivalents voire supérieurs aux mycosubtilines actuellement utilisées en tant qu'agent antifongique.

Dans la présente, divulgation on entend par « agents antifongiques », des substances possédant la capacité de traiter et/ou de prévenir des infections par des champignons et/ou des levures.

Les inventeurs ont également élaboré une composition comprenant un mélange de mycosubtilines.

Ainsi la présente invention a également pour objet une composition comprenant au moins une mycosubtiline Gln3 C17.

Par exemple, cette composition peut comprendre en outre une ou plusieurs autres mycosubtilines choisies dans le groupe comprenant une mycosubtiline *iso*-C16, une mycosubtiline n-C16, une mycosubtiline *anteiso*-C17, une mycosubtiline *iso*-C17 et une mycosuntiline C18.

Lorsqu'une mycosubtiline C18 est présente dans la composition, elle peut être présente à une concentration comprise entre 1 et 5 % en poids de la composition.

Lorsqu'une mycosubtiline Gln3 C17 est présente dans la composition, elle peut être présente à une concentration comprise entre 1 et 20 % en poids de la composition.

Lorsqu'une mycosubtiline *iso*-C16 est présente dans la composition, elle peut être présente à une concentration comprise entre 1 et 60 % en poids de la composition.

Lorsqu'une mycosubtiline n-C16 est présente dans la composition, elle peut être présente à une concentration comprise entre 1 et 10% en poids de la composition.

Lorsqu'une mycosubtiline *anteiso*-C17 est présente dans la composition, elle peut être présente à une concentration comprise entre 20 et 95 % en poids de la composition.

Lorsqu'une mycosubtiline *iso*-C17 est présente dans la composition, elle peut être présente à une concentration comprise entre 5 et 30 % en poids de la composition.

Par exemple, la composition selon l'invention peut comprendre, en pourcentage par rapport au poids de la composition : entre 1 et 60% de mycosubtiline *iso*-16, entre 1 et 20% de mycosubtiline Gln3 C17, entre 1 et 10% mycosubtiline n-C16, entre 20 et 95% de mycosubtiline *anteiso*-C17, entre 5 et 30% de mycosubtiline *iso*-C17 et entre 1 et 5% de mycosubtiline C18.

De préférence, cette composition comprend, en pourcentage par rapport au poids de la composition : 26% de mycosubtiline *iso*-16, 1% mycosubtiline Gln3 C17, 2% mycosubtiline n-C16, 44% mycosubtiline *anteiso*-C17, 23% mycosubtiline *iso*-C17 et 1% mycosubtiline C18.

Cette composition peut par exemple être utilisée en tant que composition antifongique. En d'autres termes, il peut s'agir d'une composition pour utilisation comme antifongique.

La composition comprenant un mélange de mycosubtilines selon la présente invention a un pouvoir antifongique allant d'une concentration minimale inhibitrice de 4 à 32 μM.

Les mycosubtilines et la composition comprenant un mélange de mycosubtilines selon la présente invention peuvent être mélangées dans une solution choisie dans le groupe comprenant l'eau, l'éthanol, le méthanol, le diméthylsulfoxyde (DMSO), le carbonate de sodium, le Tris-HCl et un mélange de ces solutions.

Le mélange de ces solutions peut être un mélange binaire ou ternaire. Lorsque le mélange de solutions est binaire, le ratio eau/éthanol, eau/méthanol, eau/DMSO, eau/ carbonate de sodium, eau/ Tris-HCl, éthanol/méthanol, éthanol/DMSO, éthanol/ carbonate de sodium, éthanol/ Tris-HCl, méthanol/DMSO, méthanol/ carbonate de sodium, méthanol/ Tris-HCl, peut être par exemple compris entre 4/1 et 1/4, par exemple entre 3/1 et 1/3, par exemple entre 2/1 et 1/2, par exemple un ratio de 1/1, 2/1, 3/1 ou 4/1. Lorsque le mélange de solutions est ternaire, le ratio eau/éthanol/méthanol, eau/éthanol/DMSO, eau/éthanol/carbonate de sodium, eau/éthanol/Tris-HCl, eau/méthanol/DMSO, eau/méthanol/carbonate de sodium, eau/méthanol/Tris-HCl, éthanol/méthanol/DMSO, éthanol/méthanol/carbonate de sodium, éthanol/méthanol/ Tris-HCl, DMSO/ carbonate de sodium / Tris-HCl peut être par exemple de 1/1/1, 2/1/1, 1/2/1, 1/1/2, 3/1/1, 1/3/1, 1/1/3, 3/2/1, 3/1/2, 2/3/1, 2/1/3, 1/2/3 ou 1/3/2.

Les mycosubtilines et la composition comprenant un mélange de mycosubtilines selon la présente invention peuvent par exemple être utilisées comme agent antifongique.

La présente invention a donc également pour objet, une mycosubtiline selon l'invention, ou une composition selon l'invention pour utilisation comme agent antifongique.

La présente invention fournit également une description d'une souche *Bacillus subtilis* pour produire la mycosubtiline, un procédé d'obtention de ces mycosubtilines et un dispositif permettant la production d'un biosurfactant à l'échelle industrielle, en particulier en supprimant ou limitant la formation de mousse.

Le procédé d'obtention d'un biosurfactant, comprend une étape **(a)** de culture d'un microorganisme capable de produire un biosurfactant dans un milieu de culture comprenant un substrat organique, la culture du microorganisme étant réalisée à la surface d'un contacteur membranaire air/liquide.

Les inventeurs sont en effet les tous premiers à avoir mis en oeuvre ce procédé et ont découvert, de façon surprenante, que l'immobilisation des cellules sur le contacteur membranaire air/liquide est particulièrement favorable à la production des biosurfactants en continu, tout en évitant la formation de mousse. En outre, le procédé permet d'augmenter le rendement de production de biosurfactant. De plus, l'utilisation d'un contacteur membranaire air/liquide dans le procédé selon la présente invention permet de produire un biosurfactant en continu.

Dans la présente, on entend par « biosurfactant », une molécule tensioactive qui est amphiphile et produite à partir d'un microorganisme. Il peut s'agir par exemple d'un composé choisi dans le groupe comprenant un lipopeptide, un phospholipide, un glycolipide, une lipoprotéine, un ester d'acide gras. Par exemple, le lipopeptide peut être choisi dans le groupe comprenant une iturine, une surfactine, une mycosubtiline, une syringomycine, une fengycine (ou plipastatine), une lichenysine, une bacillomycine, une kurstakine, une tolaasine, une arthrofactine, une serrawettine, une putisolvine, un massetolide. Le phospholipide peut par exemple être choisi dans le groupe comprenant une phosphatidylcholine. L'ester peut par exemple être choisi dans le groupe comprenant un ester de sorbitan, de rhamnose, une monomyristine, une monolinoléine et une monolinolénine. Le glycolipide peut par exemple être un rhamnolipide.

On entend par « culture d'un microorganisme », l'ensemble des techniques utilisées pour faire croître un microorganisme et/ou lui faire produire une ou plusieurs molécules.

On entend par « microorganisme capable de produire un biosurfactant », tout organisme microscopique unicellulaire ou pluricellulaire dénué de différenciation tissulaire, et ayant la capacité de synthétiser un biosurfactant. Il peut s'agir par exemple d'une bactérie, d'une levure, d'une moisissure ou d'une algue.

Par exemple, la bactérie peut appartenir à un genre choisi dans le groupe comprenant *Bacillus, Pseudomonas, Rhodococcus, Acinetobacter, Serratia, Burkholderia, Mycobacterium, Nocardia, Flavobacterium, Corynebacterium, Clostridium, Thiobacillus, Arthrobacter, Alcanivorax, Paenibacillus.* Par exemple, la levure peut appartenir à un genre choisi dans le groupe comprenant *Candida, Pseudozyma, Ustilago, Schizonella, Kurtzmanomyces, Torulopsis, Rhodotorula, Wickerhamiella.* De préférence, le microorganisme capable de produire un biosurfactant appartient au genre *Bacillus.*

Lorsque le microorganisme capable de produire un biosurfactant appartient au genre *Bacillus,* il peut par exemple être choisi dans le groupe comprenant *Bacillus subtilis, Bacillus thuringiensis, Bacillus licheniformis, Bacillus amyloliquefaciens, Bacillus cereus, Bacillus pumilus, Bacillus mojavensis.* Il peut s'agir par exemple des souches choisies dans le groupe comprenant *Bacillus subtilis,* telle que celle déposée le 10 mars 2011 sous le numéro CNCM I-4451 à la Collection Nationale de Cultures de Microorganismes (CNCM) de l'Institut Pasteur (Paris, France), également appelée *Bacillus subtilis* BBG125, ainsi que *Bacillus subtilis* ATCC 21332, *Bacillus subtilis* BBG21, *Bacillus subtilis* ATCC 6633, *Bacillus subtilis* BBG100, *Bacillus subtilis* ATCC 9943, *Bacillus subtilis* S499, *Bacillus subtilis* BBG116, *Bacillus subtilis* BBG131, *Bacillus licheniformis* BAS50, une souche dérivée de *Bacillus licheniformis* ATCC 14580 et *Bacillus thuringiensis* BBG300.

De préférence, pour la production de mycosubtiline, le microorganisme capable de produire un biosurfactant

est choisi dans le groupe comprenant *Bacillus subtilis* BBG125, *Bacillus subtilis* BBG100 et *Bacillus subtilis* BBG1116, de préférence dans le groupe comprenant *Bacillus subtilis* BBG125 et *Bacillus subtilis* BBG100.

De préférence, pour la production de surfactine, le microorganisme capable de produire un biosurfactant est *Bacillus subtilis* BBG131.

De préférence, pour la production de fengycine, le microorganisme capable de produire un biosurfactant est choisi dans le groupe comprenant *Bacillus subtilis* ATCC 21332, *Bacillus subtilis* BBG21.

De préférence encore, selon l'invention, le microorganisme capable de produire un biosurfactant est *Bacillus subtilis* BBG125.

Lorsque le microorganisme capable de produire un biosurfactant appartient au genre *Pseudomonas,* il peut par exemple être choisi dans le groupe comprenant *Pseudomonas aeruginosa, Pseudomonas cichorii, Pseudomonas putida, Pseudomonas fluorescens, Pseudomonas stutzeri, Pseudomonas syringae* et *Pseudomonas tolaasii.*

Dans la présente, on entend par « substrat organique », toute substance ou mélange de substances permettant de faire croître le microorganisme et/ou de lui faire produire une ou plusieurs molécules. Par exemple, le substrat organique peut être choisi dans le groupe comprenant l'amidon, le glucose, le glutamate, le saccharose, le xylose, le glycérol, les acides organiques, les acides aminés et un mélange de ces substrats organiques. Par exemple, le substrat organique peut être le milieu de Landy dont la composition est la suivante : glucose, 20 g/L ; acide glutamique, 5 g/L ; extrait de levure, 1 g/L, $K_2HPO_4$, 1 g/L ; $MgSO_4$, 0,5 g/L ; KCl, 0,5 g/L ; $CuSO_4$, 1,6 mg/L ; $Fe_2(SO_4)_3$, 1,2 mg/L ; $MnSO_4$, 0,4 mg/L (Landy et al. 1948. Bacillomycin; an antibiotic from Bacillus subtilis active against pathogenic fungi. Proc. Soc. Exp. Biol. Med. 67, 539-541) **[9]**. Le substrat organique peut également être, par exemple un milieu de Landy modifié, par exemple un milieu de Landy complété avec du sulfate d'ammonium à 2,3 g/L et/ou la concentration en acide glutamique est de 2 g/L (Guez et al., 2008. Respiration activity monitoring system (RAMOS), an efficient tool to study the influence of the oxygen transfer rate on the synthesis of lipopeptide by Bacillus subtilis. J. Biotechnol. 134, 121-126 **[10]**).

Dans la présente, on entend par « contacteur air/liquide », un dispositif permettant d'oxygéner un milieu liquide à partir d'un gaz contenant de l'oxygène. Un contacteur air/liquide comprend notamment un contacteur membranaire air/liquide. Il peut s'agir par exemple d'un réacteur comprenant un contacteur membranaire air/liquide. A titre d'exemples de contacteur air/liquide, on peut citer les contacteurs suivants : « Hollow fiber cartridges » de GE Healthcare selon les modèles définis par des références commençant par CFP et « Hollow fiber modules de Spectrum Labs » selon les modèles définis par des références commençant par KM.

Un contacteur air/liquide à membrane permet également de séparer la phase liquide de la phase gazeuse : la phase gazeuse circule d'un côté de la membrane et la phase liquide s'écoule de l'autre côté (Remize et Cabassud. 2003 Un nouveau réacteur d'oxydation sans bulles : le contacteur à membrane G/L. Récents progrès en génie des procédés. Intégration des membranes dans les procédés 2. Lavoisier Tec et Doc. **[11]**), sans que ces deux phases ne se mélangent.

On entend par « contacteur membranaire air/liquide », une membrane poreuse permettant la diffusion d'oxygène dans un milieu de culture.

Par exemple, le contacteur membranaire air/liquide peut être une membrane en fibres creuses ou en membranes planes.

Le contacteur membranaire air/liquide peut par exemple posséder des pores dont la taille est comprise entre 0,01 et 2 $\mu$m, par exemple entre 0,01 à 1 $\mu$m, par exemple entre 0,1 et 0,65 $\mu$m.

Le contacteur membranaire air/liquide peut par exemple posséder une surface comprise entre 0,1 et 20 $m^2$. De préférence, la surface du contacteur membranaire air/liquide est supérieure à 1 $m^2$.

Le contacteur membranaire air/liquide peut par exemple être une membrane hydrophobe, permettant de garantir une meilleure séparation de ces deux phases. Par exemple, le contacteur membranaire air/liquide peut être réalisé dans un matériau choisi dans le groupe de polymères comprenant le polyéthersulfone, le polypropylène, le polysulfone, la cellulose régénérée et les esters de cellulose.

Le contacteur membranaire air/liquide peut être par exemple plan, cylindrique, cylindroconique ou de toute forme géométrique permettant d'optimiser la surface de culture de microorganisme et les échanges avec l'oxygène.

A titre d'exemple de contacteurs membranaires air/liquide, on peut citer notamment les membranes suivantes :

- (CFP-6-D-45) de GE-Healthcare Europe GmbH (Munich, Allemagne),
- modules de filtration à fibres creuses (Hollow fiber cartridges) de GE Healthcare selon les modèles définis par des références commençant par CFP,
- modules de filtration à fibres creuses (Hollow fiber modules) de Spectrum Labs selon les modèles définis par des références commençant par KM.

Les microorganismes capables de produire un biosurfactant peuvent être immobilisés activement soit totalement soit partiellement à la surface du contacteur membranaire air/liquide. En d'autres termes, une grande partie des

microorganismes présents dans le contacteur air/liquide sont immobilisés à la surface de la membrane de ce contacteur, l'autre partie se trouvant en suspension dans le milieu de culture après leur relargage.

Ainsi, le procédé peut comprendre une étape **(a)** de culture d'un microorganisme capable de produire un biosurfactant dans un milieu de culture comprenant un substrat organique, la culture du microorganisme étant réalisée à la surface d'un contacteur membranaire air/liquide. En d'autres termes, le procédé permet avantageusement de se passer d'une cuve de culture ou d'un fermenteur. Le procédé permet avantageusement de produire des mycosubtilines en continu et en quantité très satisfaisante. Avantageusement, le procédé est réalisé en continu. Une cuve de culture ou un fermenteur peut être ajouté éventuellement mais n'est pas indispensable. L'ajout d'une cuve de culture ou d'un fermenteur est plutôt déconseillé car il diminuerait les rendements de production. Ainsi, avantageusement, le procédé est mis en oeuvre dans un dispositif ne comprenant pas de cuve de culture ou un fermenteur. En d'autres termes, selon le procédé, la culture du microorganisme capable de produire un biosurfactant peut être réalisée à la surface le contacteur membranaire air/liquide uniquement.

L'oxygène nécessaire aux microorganismes capables de produire un biosurfactant est transféré par diffusion à travers les pores du contacteur membranaire air/liquide où sont immobilisés lesdits microorganismes. En d'autres termes, l'oxygénation du milieu des microorganismes n'est pas réalisée par un système de bullage situé dans un réacteur ni par un système d'oxygénation situé à l'extérieur du réacteur de culture des microorganismes.

Le débit d'oxygène du contacteur membranaire air/liquide peut être ajusté à tout débit permettant d'oxygéner les microorganismes capables de produire un biosurfactant. L'homme du métier est à même de déterminer les débits d'oxygène du contacteur membranaire air/liquide en fonction de l'apport d'oxygène souhaité. Les inventeurs ont constaté qu'un débit d'aération compris entre 0,2 et 2 volumes d'air par volume de liquide par minute (vvm) est particulièrement efficace pour oxygéner le milieu de culture et les microorganismes. Le débit d'air du contacteur air liquide peut donc être par exemple compris entre 0,5 et 2 vvm. De préférence, le débit d'aération du contacteur air/liquide est de 0,25 vvm pour la production de mycosubtiline. De préférence, le débit d'aération du contacteur air/liquide est de 1 vvm pour la production de surfactine. De préférence, le débit d'aération du contacteur air/liquide est de 0,5 vvm pour la production de fengycine.

Selon le procédé, l'étape **(a)** de culture peut être réalisée à la surface d'une pluralité de contacteurs membranaires air/liquide. Par exemple, l'étape **(a)** de culture peut être réalisée à la surface de 2 contacteurs membranaires air/liquide, par exemple 3, 4, 5, 6, 7, 8, 9, 10 membranes air/liquide, voire plus. L'homme du métier est à même de déterminer le nombre de contacteurs membranaires air/liquide en fonction de la quantité de biosurfactant à produire.

Un des objectifs est d'augmenter la quantité de microorganismes immobilisés à la surface du contacteur membranaire air/liquide. Cela peut être réalisé par une augmentation de la surface de contacteur membranaire air/liquide et/ou du nombre de contacteurs membranaires air/liquide.

Lorsque l'étape **(a)** de culture est réalisée à la surface d'une pluralité de contacteurs membranaires air/liquide, les contacteurs membranaires air/liquide peuvent par exemple être disposés en série ou en parallèle. De préférence, lorsque l'étape **(a)** de culture est réalisée à la surface d'une pluralité de contacteurs membranaires air/liquide, les contacteurs membranaires air/liquide sont disposés en parallèle.

Le procédé peut comporter en outre une étape de séparation du biosurfactant du milieu de culture le contenant. Cette étape de séparation peut être réalisée par tout moyen connu de l'homme du métier permettant de séparer une substance contenue dans un milieu liquide de celui-ci.

Par exemple, l'étape de séparation du biosurfactant du milieu de culture le contenant peut comprendre une ou plusieurs étapes, choisies dans le groupe comprenant une microfiltration, une ultrafiltration, une nanofiltration et une centrifugation.

Par exemple, l'étape de séparation du biosurfactant du milieu de culture le contenant comprend les étapes suivantes :

**(b)** microfiltration du milieu de culture obtenu à l'étape **(a),** permettant de séparer le microorganisme du milieu de culture, et/ou

**(c)** ultrafiltration du milieu de culture obtenu à l'étape **(a)** ou **(b),** permettant de séparer le biosurfactant du milieu de culture obtenu à l'étape **(a)** ou **(b).**

De préférence, l'étape de séparation comprend chacune des étapes **(b)** et **(c).**

Les étapes **(b)** de microfiltration et **(c)** d'ultrafiltration permettent d'extraire en continu le biosurfactant du milieu de culture obtenu à l'étape **(a)** et/ou **(b).**

Ainsi, la combinaison du contacteur membranaire air/liquide avec les étapes **(b)** de microfiltration et **(c)** d'ultrafiltration permet de produire et d'extraire en continu un biosurfactant à partir d'un microorganisme capable de le produire.

L'étape **(b)** de microfiltration peut être réalisée avec tout moyen de microfiltration permettant de séparer le microorganisme du milieu de culture le contenant. Par exemple, le moyen de microfiltration peut être une membrane

de microfiltration. Par exemple, le moyen de microfiltration peut être une membrane de microfiltration organique ou minérale, par exemple une membrane en fibres creuses.

L'étape **(b)** de microfiltration peut par exemple être réalisée avec une membrane en fibres creuses possédant des tailles de pores de 0,1 à 0,45 micromètre (μm). De préférence, la membrane en fibres creuses utilisée à l'étape **(b)** possède une taille de pores de 0,2 μm.

A titre d'exemple de membranes utilisables pour réaliser l'étape **(b)** de microfiltration on peut citer les membranes suivantes :

- fibres creuses de polysulfone ou de polyéthersulfone de taille de pores 0,2 μm de référence CFP-2-E-4X2MA (GE-Healthcare Europe GmbH, Munich, Allemagne),
- fibres creuses de polysulfone ou de polyéthersulfone de taille de pores 0,45 μm de référence CFP-4-E-4X2MA ou de taille de pore 0,65 μm de référence CFP-2-E-6X2MA (GE-Healthcare Europe GmbH, Munich, Allemagne),
- module de microfiltration ou d'ultrafiltration à fibres creuses (Hollow fiber cartridges) de GE Healthcare selon les modèles définis par des références commençant par CFP,
- module de filtration à fibres creuses (Hollow fiber modules) de Spectrum Labs (Rancho Dominguez, CA, U.S.A.) selon les modèles définis par des références commençant par KM,
- cassettes Sartocon de microfiltration de Sartorius Stedim (Aubagne, France) selon les modèles définis par des références commençant par SPC20.

L'étape **(c)** d'ultrafiltration peut être réalisée avec tout moyen d'ultrafiltration permettant de séparer le biosurfactant du milieu de culture le contenant, et de concentrer le biosurfactant. Par exemple, le moyen d'ultrafiltration peut être une membrane d'ultrafiltration, par exemple une membrane d'ultrafiltration en cellulose régénérée.

L'étape **(c)** d'ultrafiltration peut par exemple être réalisée avec une membrane possédant un seuil de coupure compris entre 5 et 50 kilodaltons (kDa), par exemple entre 5 kDa et 30 kDa, par exemple entre 5 et 20 kDa. De préférence, la membrane utilisée à l'étape **(c)** possède un seuil de coupure de 10 kDa.

A titre d'exemple de membranes utilisables pour réaliser l'étape **(c)** d'ultrafiltration, on peut citer les membranes suivantes :

- Membrane d'ultrafiltration de seuil de coupure de 10 KDa en cellulose régénérée de référence 3051443901E-SW (Sartorius, Göttingen, Allemagne),
- Membrane d'ultrafiltration de seuil de coupure de 10 kDa en cellulose régénérée de référence P2C010C01 (Millipore Headquarters, 290 Concord Road, Billerica, MA, U.S.A.).

De préférence, les membranes utilisées aux étapes **(a), (b)** et **(c)** sont stérilisables à 121°C pendant 20 minutes.

Le procédé se différencie des procédés connus de dégradation biologique de matières organiques par des microorganismes qui excrètent des biosurfactants en ce que l'on peut recycler ou non la totalité des microorganismes après les avoir débarrassés des résidus de matières organiques du milieu de culture et des biosurfactants produits. Cela permet d'obtenir un taux élevé de concentration des microorganismes dans le bioréacteur.

Il se différencie également des procédés connus de dégradation biologique de matières organiques par des microorganismes qui excrètent des biosurfactants en ce qu'environ 95% des biosurfactants produits restent dans le milieu de culture sans la moindre formation de mousse. Environ 5% des biosurfactants sont adsorbés à l'interface air/liquide du contacteur à membrane, mais peuvent être désorbés, par exemple par un lavage de la membrane.

Le contacteur membranaire air/liquide peut être lavé par tout moyen connu de l'homme du métier afin de récupérer les biosurfactants produits et adsorbés à l'interface air/liquide du contacteur à membrane. Par exemple, le lavage du contacteur membranaire air/liquide peut être réalisé avec une ou plusieurs solutions de lavage choisies dans le groupe comprenant de l'eau distillée, une solution de NaOH, une solution de NaOCl, une solution d'hydrogénocarbonate de sodium ou de potassium, une solution de carbonate de sodium ou de potassium. La solution de lavage peut être amenée à tout pH permettant d'augmenter la quantité de biosurfactants récupérés. Par exemple, la solution de lavage est amenée à un pH = 10.

La solution de lavage peut être amenée à toute température permettant d'augmenter la quantité de biosurfactants récupérés. Par exemple, la solution de lavage est amenée à une température comprise entre 20 et 50°C.

La température du milieu de culture peut être réglée par tout moyen de chauffage connu de l'homme du métier. Par exemple, le moyen de chauffage peut être un échangeur thermique. Par exemple, l'échangeur thermique peut être choisi dans le groupe comprenant un échangeur thermique à tube en U, un échangeur thermique à faisceau tubulaire horizontal, un échangeur thermique à faisceau tubulaire vertical, un échangeur thermique à spirales, un échangeur thermique à plaques, une colonne Bouhy, un échangeur thermique à bloc, De préférence, le moyen de chauffage est un échangeur thermique tubulaire.

Ainsi, le procédé peut être mis en oeuvre à toute température permettant de produire un biosurfactant à partir

d'un microorganisme capable de le produire. Par exemple, le procédé peut être mis en oeuvre à une température comprise entre 0°C et 70°C, avantageusement entre 20°C et 37°C. De préférence, le procédé peut être mis en oeuvre à une température de 22°C pour la production de mycosubtiline. De préférence, le procédé peut être mis en oeuvre à une température de 30°C pour la production de fengycine. De préférence, le procédé peut être mis en oeuvre à une température de 37°C pour la production de surfactine.

Par ailleurs, le procédé peut être mis en oeuvre à tout pH permettant de produire un biosurfactant à partir d'un microorganisme capable de le produire. Le pH peut être régulé grâce à l'ajout contrôlé de solution basique ou de solution acide dans le milieu de culture.

La solution basique peut, par exemple, être choisie dans le groupe comprenant la soude, la potasse et l'ammoniaque.

La solution acide peut, par exemple, être choisie dans le groupe comprenant l'acide phosphorique, l'acide sulfurique et l'acide nitrique.

Le pH peut par exemple être régulé à toute valeur permettant la survie des microorganismes capables de produire un biosurfactant. Il peut par exemple être régulé à une valeur comprise entre pH 6 et pH 8, de préférence à une valeur de pH 7. L'homme du métier est à même de déterminer les quantités de solution basique et de solution acide pour réguler le pH à une valeur souhaitée.

Le procédé peut avantageusement être réalisé en continu, c'est-à-dire que l'alimentation du contacteur membranaire air/liquide et l'extraction des biosurfactants produits par les microorganismes peuvent être réalisées sans interruption. Le procédé peut être réalisé à tout débit horaire permettant d'extraire un biosurfactant à partir d'un microorganisme capable de le produire. Le débit auquel peut être mis en oeuvre le procédé, c'est-à-dire le débit de milieu de culture ajouté dans le contacteur membranaire air/liquide peut aisément être adapté par l'homme du métier. Les inventeurs ont constaté que la conduite du procédé continu à un taux de dilution entre 0,05 $h^{-1}$ et 0,5 $h^{-1}$ est particulièrement efficace pour produire des biosurfactants à partir de microorganismes. Le taux de dilution est défini comme le quotient du débit d'alimentation ou d'extraction sur le volume de culture. Le procédé peut donc, par exemple, être réalisé à un débit horaire circulant correspondant à un taux de dilution compris entre 0,05 $h^{-1}$ et 0,5 $h^{-1}$, avantageusement à un débit horaire de 0,1 $h^{-1}$.

Le dispositif pour la mise en oeuvre du procédé décrit dans la présentecomporte un contacteur membranaire air/liquide. Par exemple, le dispositif comprend au moins un contacteur air/liquide comprenant un contacteur membranaire air/liquide.

Le contacteur membranaire air/liquide et le contacteur air/liquide peuvent être ceux définis ci-dessus.

Le nombre de contacteurs membranaires air/liquide et de contacteurs air/liquide peut être comme défini ci-dessus.

Le dispositif ne comprend pas de moyen d'aération autre que la membrane ou la pluralité de contacteur membranaire air/liquide. En d'autres termes, le dispositif ne comprend pas un système de bullage situé dans un réacteur. Il ne comprend pas non plus un système d'oxygénation situé à l'extérieur du réacteur de culture des microorganismes. Le dispositif peut en outre comprendre un moyen de microfiltration et/ou un moyen d'ultrafiltration. De préférence, le dispositif comprend un moyen de microfiltration et un moyen d'ultrafiltration. Le moyen de microfiltration et le moyen d'ultrafiltration peuvent par exemple être ceux décrits ci-dessus.

Avantageusement, le dispositif comprend les moyens nécessaires pour la mise en oeuvre en continu du procédé décrit. En d'autres termes, le dispositif comprend avantageusement un moyen d'introduction de milieu de culture et un moyen de prélèvement du biosurfactant produit par le microorganisme. Tout moyen d'introduction et tout moyen de prélèvement connus de l'homme du métier pour obtenir un dispositif permettant la mise en oeuvre en continu du procédé décrit peuvent être utilisés.

Le dispositif peut en outre comprendre un moyen d'évaporation. Dans la présente, on entend par « moyen d'évaporation » tout moyen permettant de concentrer un biosurfactant dans un milieu le contenant. Il peut s'agir par exemple d'un moyen d'évaporation choisi dans le groupe comprenant un évaporateur sous vide de type Rotavapor VV2000 (Heidolph Instruments GmbH & Co, Schwabach, Allemagne) et un évaporateur à film grimpant. Le dispositif peut en outre comprendre un moyen de chauffage permettant de régler la température du milieu de culture. Le moyen de chauffage peut par exemple être celui décrit ci-dessus. Le système de chauffage peut être relié au contacteur air/liquide via un système de canalisations.

On entend par « système de canalisations », tout moyen dans lequel peut circuler un fluide ou un gaz. Par exemple le fluide peut être un liquide ou un gel. Le système de canalisations permet notamment de relier entre eux, différents éléments du dispositif selon l'invention. Par exemple, le système de canalisations peut être tout type de tuyauterie flexible ou rigide de type silicone (Cole Parmer, Vernon Hills, IL, U.S.A.) ou en acier inoxydable 316S (Swagelok Company, Solon, OH, U.S.A.).

La circulation du fluide ou gaz dans le système de canalisation peut être régulée par une ou plusieurs pompes et/ou une ou plusieurs vannes.

Le dispositif peut en outre comprendre au moins une pompe.

On entend par « pompe » au sens de la présente invention, un moyen permettant d'imposer un débit à un liquide, par exemple à un milieu de culture dans le dispositif. Il peut par exemple s'agir d'une pompe péristaltique, d'une pompe à lobes ou d'une pompe à membrane. On peut citer par exemple, les pompes péristaltiques Masterflex L/S compact drive model (Cole Parmer Vernon Hills, IL, U.S.A.), Millipore Corporation (Millipore, Bedford, MA, U.S.A.), Sartojet pump (Sartorius, Sartorius Stedim France SAS, Aubagne) et Watson-Marlow 323 (Watson Marlow, Falmouth, Cornwall, Royaume-Uni). Par ailleurs, la pompe peut être commandée manuellement ou automatiquement.

Le dispositif peut en outre comprendre au moins une vanne.

Dans la présente, on entend par « vanne », un moyen permettant d'arrêter ou de modifier le débit d'un liquide, par exemple du milieu de culture dans le dispositif de la présente invention. Il peut par exemple s'agir d'une vanne de régulation, d'une vanne « tout ou rien », d'une électrovanne. On peut citer par exemple, les vannes de régulation en fluorure de polyvinylidène (PVDF), en polypropylène (PP), en perfluoroalkoxy (PFA) ou en acier inoxydable. Par ailleurs, la vanne peut être commandée manuellement ou automatiquement.

Les inventeurs décrivent dans la présente l'utilisation du dispositif pour la mise en oeuvre du procédé décrit.

La présente divulgation décrit également une souche *Bacillus subtilis* obtenue à partir de la souche *Bacillus subtilis* ATTC 6633 dans laquelle l'opéron *srfA* codant pour la surfactine synthétase a été interrompu et dont le promoteur de l'opéron *myc* codant pour la mycosubtiline synthétase a été remplacé par un promoteur fort constitutif $P_{repU}$.

De préférence, cette souche *Bacillus subtilis* est la souche *Bacillus subtilis* déposée le 10 mars 2011 sous le numéro CNCM I-4451 à la Collection Nationale de Cultures de Microorganismes (CNCM) de l'Institut Pasteur (Paris, France). Cette souche est également appelée *Bacillus subtilis* BBG125.

Le procédé de production de mycosubtilines comprenant une étape de culture d'une souche de *Bacillus subtilis* et une étape de récupération des mycosubtilines obtenues.

La souche *Bacillus subtilis* BBG125 peut par exemple être utilisée dans tout procédé de production de biosurfactants, notamment dans un procédé de production de mycosubtilines C18 et Gln3 C17 décrites ci-dessus. Par exemple, le procédé de production de biosurfactants peut comprendre une étape de culture de la souche *Bacillus subtilis* BBG125 et une étape de récupération des biosurfactants obtenus. Par exemple, le procédé de production de biosurfactants peut être celui de la présente décrit ci-dessus.

La souche *Bacillus subtilis* BBG125 développée par les inventeurs est particulièrement surprenante. Cette souche permet de produire des mycosubtilines sans production de surfactine. En outre, elle permet de produire des mycosubtilines qui n'ont jamais été décrites.

Les inventeurs fournissent donc un procédé de production en continu de biosurfactants permettant d'éviter la formation de mousse et d'augmenter les rendements de production. Ils ont également fourni un dispositif permettant de mettre en oeuvre ce procédé ainsi qu'une souche de *Bacillus subtilis* remarquable en ce qu'elle est capable de produire dans ce procédé des mycosubtilines qui n'ont jamais été décrites et qui présentent des effets antifongiques équivalents voire supérieurs aux mycosubtilines actuellement utilisées en tant qu'agent antifongique. Les inventeurs ont également fourni une composition antifongique présentant des effets antifongiques supérieurs aux effets des mycosubtilines actuellement utilisées.

D'autres avantages pourront encore apparaître à l'homme du métier à la lecture des exemples ci-dessous, illustrés par les figures annexées, données à titre illustratif.

## Brève description des figures

- La figure 1 est un schéma représentant un dispositif de production en continu sans formation de mousse et d'extraction des lipopeptides produits par un microorganisme. Sur cette figure, M1 représente un contacteur air/liquide à membrane en fibres creuses possédant les compartiments C1 et C2 représentant respectivement un compartiment externe, dans lequel circule de l'air, et un compartiment interne, dans lequel circule un milieu de culture. M2 représente la membrane de microfiltration d'un moyen de microfiltration possédant les compartiments C3 et C4. M3 représente la membrane d'ultrafiltration d'un moyen d'ultrafiltration possédant les compartiments C5 et C6. B1, B2 et B3 représentent chacune une balance. B représente un moteur permettant d'animer un moyen d'agitation dans la Cuve 2. P1, P2, P3, P4, P5, P6 et P7 représentent des pompes volumétriques. « Mi » signifie « milieu de culture », « De » signifie « déchets » et « Et » signifie « échangeur thermique ».
- La figure 2 est un schéma représentant un dispositif de production en continu sans formation de mousse, d'extraction et de purification des lipopeptides produits par un microorganisme. Sur cette figure, C1, C2, C3, C4, C5, C6, M1, M2, M3, B1, B2, B3, P1, P2, P3, P4, P5, P6, P7, « Mi », « De » et « Et » ont la même signification que pour la figure 1. M4 représente la membrane d'ultrafiltration d'un moyen d'ultrafiltration possédant les compartiments C7 et C8. B4, B5, B6 et B7 représentent chacune une balance. B représente un moteur permettant d'animer un moyen d'agitation dans la Cuve 2 ou de la Cuve 4. P8, P9, P10, P11 et P12 représentent des pompes volumétriques. V1,

V2, V3, V4 et V5 représentent des vannes. « Co » signifie « condenseur » et « X » correspondant à une solution liquide comprenant des lipopeptides produits par le microorganisme.

- La figure 3 est un schéma représentant le dispositif de la figure 1 dans lequel un circuit alternatif est représenté en pointillés. P13 et P14 représentent des pompes volumétriques. V6, V7 et V8 représentent des vannes. B7 représente une balance.
- La figure 4 est un schéma représentant le dispositif de la figure 2 dans lequel un circuit alternatif est représenté en pointillés. P13 et P14 représentent des pompes volumétriques. V6, V7 et V8 représentent des vannes. B7 représente une balance. La figure 5 représente les premières parties du dispositif décrit dans les figures 1 à 4, dans lequel il y a une pluralité de contacteurs air/liquide qui sont disposés en parallèle. Sur ce schéma, trois contacteurs air/liquide sont représentés.
- La figure 6 est une représentation schématique de la recombinaison homologue d'un fragment de 2,6 kb, portant la séquence ε*pbp*-$P_{repU}$-*neo*-ε*fenF* du plasmide pBG106 dont les fragments ε*pbp* et ε*fenF* sont issus par PCR de *Bacillus subtilis* ATCC 6633, formant ainsi le plasmide pBG200. Sur cette figure, « *Sph*I », « *Xba*I », « *Bsp*EI » et « *Xma*I » représentent les sites de restriction des enzymes éponymes respectives. « ε*pbp* » et « ε*fenF* » représentent des cassettes permettant la recombinaison homologue. « *pbp* » représente le gène codant pour une protéine se liant à la pénicilline. « $P_{myc}$ » représente le promoteur originel de *B. subtilis* ATCC 6633. « *fenF* », « *mycA* », « *mycB* » et « *mycC* » représentent les quatre gènes qui constituent l'opéron de la mycosubtiline. « *yngL* » représente le gène codant pour une protéine ayant une fonction inconnue. « $P_{repU}$ » représente le promoteur du gène de réplication de pUB110. « *neo* » représente un gène conférant une résistance à la néomycine/kanamycine.
- La figure 7 est une représentation schématique de la recombinaison homologue d'un plasmide pBG144 au niveau de l'opéron *srfA* de la souche BBG116, aboutissant à la souche BBG125. « *Eco*RI », « *Bst*EII » et « *Mfe*I » représentent les sites de restriction des enzymes éponymes respectives. « ε*srfAA* » et « ε*srfAA'* » représentent des cassettes permettant la recombinaison homologue. « *hxlR* » représente un gène situé en amont de l'opéron *srfA*. « $P_{srfA}$ » représente le promoteur natif de l'opéron *srfA*. « *cat* » représente un gène de résistance au chloramphénicol. « *tet* » représente un gène de résistance à la tétracycline.

## EXEMPLES

### Exemple 1 : Construction de la souche *Bacillus subtilis* BBG125

La souche *Bacillus subtilis* BBG125 a été déposée le 10 mars 2011 sous le numéro CNCM I-4451 à la Collection Nationale de Cultures de Microorganismes (CNCM) de l'Institut Pasteur (Paris, France).

Elle a été construite à partir de la souche *Bacillus subtilis* du type sauvage ATCC 6633 (Duitman et al., 1999. The mycosubtilin synthetase of Bacillus subtilis ATCC 6633: a multifunctional hybrid between a peptide synthetase, an amino transferase, and a fatty acid synthase. Proc. Natl. Acad. Sci. U.S.A., 96, 13294-13299 **[12]**) selon le protocole décrit ci-dessous.

<u>1.1 Protocole de construction du plasmide hybride pBG200 contenant ε*pbp*-$P_{repU}$-*neo*-ε*fenF* et *rep*(R6K).</u>

Le plasmide pBG106 (Leclère et al., 2005. Mycosubtilin overproduction by Bacillus subtilis BBG100 enhances the organism's antagonistic and biocontrol activities. Appl. Environ. Microbiol., 71, 4577-4584 **[13]**), a été digéré par les enzymes de restriction *Sph*I (Fermentas, Villebon sur Yvette, France ; référence ER0601) et SacI (Fermentas, Villebon sur Yvette, France ; référence ER1131) afin d'isoler et purifier un fragment ε*pbp*-$P_{repU}$-*neo*-ε*fenF* de 2,6 kilo-paires de bases (kb) de séquence SEQ ID NO: 11, selon le protocole décrit dans : Sambrook and Russell, 2001. Molecular cloning: a laboratory manual, 3rd ed., Cold Spring Harbor Laboratory, Cold Spring Harbor, New York **[14].**

Cette séquence porte deux cassettes (ε*pbp* et ε*fenF*) permettant la réalisation de recombinaisons homologues avec le chromosome de la souche *Bacillus subtilis* ATCC 6633.

Parallèlement, le plasposon pTnMod-RKm' (Dennis and Zylstra, 1998. Plasposons: modular self-cloning minitransposon derivatives for rapid genetic analysis of gram-negative bacterial genomes. Appl. Environ. Microbiol. 64, 2710-2715 **[15]**) a été traité par les enzymes de restriction *NspI* (Fermentas, Villebon sur Yvette, France ; référence ER1471) et SacI (Fermentas, Villebon sur Yvette, France ; référence ER1131) générant un mélange de cinq fragments, parmi lesquels le fragment portant *rep*(R6K) de 451 paires de bases (pb) dont la séquence a été isolée et purifiée (Sambrook and Russell, 2001 **[14]**).

Les fragments contenant les séquences ε*pbp*-$P_{repU}$-*neo*-ε*fenF* et rep(R6K) ont ensuite été ligaturés ensemble (Sambrook and Russell, 2001 **[14]**).

Les sites des enzymes de restriction utilisés ont été les suivants :

- *SphI* : GCATGC (en position 1 de la séquence SEQ ID NO: 11),

- *XbaI* : TCTAGA (en positions 743 et 2088 de la séquence SEQ ID NO: 11) et
- *SacI* : GAGCTC ((en position 2630 de la séquence SEQ ID NO: 11).

Les cassettes *εpbp,* $P_{repU}$-*neo* et ε*fenF* ont été composé de la manière suivante :

- Cassette *εpbp :* de *SphI* à *XbaI* (SEQ ID NO: 12),
- Cassette $P_{repU}$-*neo* : de *XbaI* à *XbaI* (SEQ ID NO: 13)
- Cassette *εfenF* : de *XbaI* à *SacI* (SEQ ID NO: 14).

La ligature obtenue a été utilisée pour transformer la souche d'*Escherichia coli* CC118(λ*pir*) (Herrero, de Lorenzo and Timmis, 1990. Transposon vectors containing non-antibiotic resistance selection markers for cloning and stable chromosomal insertion of foreign genes in gram-negative bacteria. J. Bacteriol. 172: 6557-67 **[16]**), avec une sélection sur milieu Luria-Bertani (ou milieu LB ou Luria Broth) (Bertani, 2004. Lysogeny at mid-twentieth century: P1, P2, and other experimental systems. J. Bacteriol. 186, 595-600 **[17]**) contenant 20 μg/mL de néomycine. Le plasmide obtenu chez *E. coli* CC118(λ*pir*), a été nommé pBG200 (3,1 kb).

La figure 6 représente schématiquement cette construction.

1.2 Protocole d'obtention de BBG116.

La souche *B. subtilis* RFB102 (souche dérivée de *B. subtilis* ATCC 6633 obtenue par insertion de la cassette *Pspac-comK* dans *amyE.* P*spac* désigne le promoteur issu du plasmide pA-spac (Bacillus Genetic Stock Center, Columbus, OH, U.S.A.) inductible par l'IPTG, *comK* désigne un gène essentiel pour la compétence naturelle chez *Bacillus.* Il est associé à un gène de résistance à la spectinomycine (P*spac-comK-spc*), qui est intégré dans le gène chromosomique *amyE.* La souche RFB102 présente une aptitude accrue pour la transformation par compétence naturelle, qui est induite par l'IPTG (isopropyl-β-D-galactopyranoside). Elle a été transformée par pBG200 préalablement traité par le système d'amplification plasmidique TempliPhi (GE Healthcare), puis sélectionnée grâce à la résistance à la néomycine, selon le protocole décrit dans Dubnau, 1982 (Genetic transformation of Bacillus subtilis p148-178. In D. Dubnau (Ed) The molecular biology of the Bacilli, vol I. Bacillus subtilis. Academic Press, Inc. New York **[18]**).

Parmi les clones Nm-R, l'insertion par double crossing-over de la cassette ε*pbp*-$P_{repU}$-*neo-εfenF* dans le chromosome de RFB102, a été vérifiée par PCR à l'aide des amorces PBP-FO2: AATAACGGACATGCCGAAGTG (SEQ ID NO: 1) et FENF-REV2: AATAGGCCGACCAAGACGTTC (SEQ ID NO : 2).

La surproduction de mycosubtiline en milieu de Landy/MOPS à 22°C a été vérifiée selon le mode opératoire décrit dans l'exemple 2 ci-dessous.

La souche *B. subtilis* BBG116 a ainsi été obtenue.

1.3 Protocole de construction du plasmide pBG144

Un plasmide pBG212 de 6,5 kb dédié à l'inactivation insertionnelle («knock-out») de l'opéron *srfA* de *B. subtilis* a été construit de la manière suivante :

Une cassette ε*srfAA* (2,2 kb) a été générée par PCR à l'aide des amorces SRF-FO ACAGGAATATGCTCAATCGAAG (SEQ ID NO : 3) et SRF-REV AAATTCGCTTCCAGGCTTCTG (SEQ ID NO : 4), à partir de l'ADN génomique de *B. subtilis* subsp. *subtilis* souche 168 (Accession NCBI PRJNA76) préalablement insérée dans le plasmide pGEM-T Easy (Promega Corp., Charbonnières, France).

Cet amplicon a été par la suite sous-cloné dans le site *Eco*RI (Fermentas, Villebon sur Yvette, France ; référence ER0271) du vecteur pUC19 (New England Biolabs, Ipswich, MA, U.S.A.).

La cassette ε*srfAA* a été ensuite interrompue au site *Mfe*I (Fermentas référence ER0751) par l'insertion du gène *tet,* préalablement généré par PCR à l'aide des amorces TETP1 GTTGTATCGATGATGAAATACTGAATTTTAAACTTAG (SEQ ID NO: 5) et TETT1 TTTAATGGATCTAGAAGATTTGAATTCCTGTTAT (SEQ ID NO: 6), à partir du plasmide pBC16 (DSMZ GmbH, Braunschweig, Allemagne), originaire de *Bacillus cereus* (Accession : NC_001705.1).

Le plasmide pBG144 a été obtenu par insertion, au site *BstE*II (Fermentas, Villebon sur Yvette, France ; référence ER0391) situé à la fin du gène *tet,* du gène *cat* préalablement généré par PCR à l'aide des amorces pC194cmfwd AGAAAGCAGACAGGTAACCCTCCTAA (SEQ ID NO : 7) et pC194cmrev GCAGGTTAGTGACATTAGGTAACCGA (SEQ ID NO : 8) du plasmide pC194 originaire de *Staphylococcus aureus* (DSMZ GmbH, Braunschweig, Allemagne, Accession : NC_002013.1).

La figure 7 représente schématiquement cette construction.

1.4 Protocole d'obtention de *B. subtilis* BBG125

De nouvelles transformations de *B. subtilis* BBG116 avec le plasmide pBG144 préalablement linéarisé par *Aat*II (Fermentas, Villebon sur Yvette, France ; référence ER0991) selon le protocole décrit dans Dubnau, 1982 (Genetic transformation of Bacillus subtilis p148-178. In D.A. Dubnau (Ed) The molecular biology of the Bacilli, vol I. Bacillus subtilis. Academic Press, Inc. New York **[18]**).

Six clones Cm-R Tc-R ont été isolés sur milieu LB gélosé contenant les antibiotiques appropriés. Des vérifications par PCR ont été réalisées à l'aide des amorces SRFAA5-FWD ; AAGGAATCTCGCAATCATTTATCG (SEQ ID NO : 9) et SRFAA5REV ; CTTGGTGTAAGCGGAATTTCTGTC (SEQ ID NO : 10). La non-production de surfactine en milieu de Landy/MOPS à 37°C a été vérifiée selon le mode opératoire décrit dans l'exemple 2 ci-dessous.

Le mutant *B. subtilis* BBG125 a été retenu comme souche monoproductrice de mycosubtiline.

Les deux souches *B. subtilis* BBG116 et BBG125 présentent des activités hémolytiques sur gélose contenant 5% de sang ainsi que des activités antifongiques sur levures et moisissures sur milieu PDA.

Ces activités sont plus marquées dans le cas de la souche *B. subtilis* BBG116, du fait de la synergie entre la surfactine produite et une surproduction de mycosubtilines. Ces propriétés sont en relation étroite avec leur capacité à coloniser la surface des milieux gélosés, grâce à l'abaissement de leur tension de surface.

Les caractéristiques résumées de *B. subtilis* BBG125 et de ses souches parentales sont présentées dans le tableau 1 ci-dessous :

**Tableau 1 : Caractéristiques résumées de *B. subtilis* BBG125 et de ses souches parentales**

| ***B. subtilis*** | **Souche-mère** | **Génotype** | **Phénotype** |
|---|---|---|---|
| **ATCC 6633** | - | Sauvage | $Com^-$ $Myc^+$ $Srf^+$ |
| **RFB102** | ATCC 6633 | *amyE::Pspac-comK-spc* | $Com^{++}$ $Amy^-$ $Myc^+$ $Srf^+$ $Spc^R$ |
| **BBG116** | RFB102 | *amyE::Pspac-comK-spc, myc::*$P_{repU}$*-neo* | $Com^{++}$ $Amy^-$ $Myc^{+++}$ $Srf^+$ $Spc^R$ $Nm^R$ |
| **BBG125** | BBG116 | *amyE::Pspac-comK-spc, myc::*$P_{repU}$*-neo, srfAA:: at-tet* | $Com^{++}$ $Amy^-$ $Myc^{++}$ $Srf^-$ $Spc^R$ $Nm^R$ $Tc^R$ $Cm^R$ |

[Com : compétence naturelle pour la transformation; Myc: production de mycosubtiline; Srf: production de surfactine; Amy: activité amylolytique ; $Spc^R$, $Nm^R$, $Tc^R$, $Cm^R$ : résistances à la spectinomycine, la néomycine/kanamycine, la tétracycline et au chloramphénicol, respectivement.]

En comparaison avec la souche *Bacillus subtilis* ATCC 6633, la souche *Bacillus subtilis* BBG125 produit une quantité plus importante de mycosubtilines et ne produit pas de surfactine.

**Exemple 2 : Préparation des milieux de culture**

2.1 Le milieu de Landy

La composition du milieu de Landy est la suivante : glucose, 20 g/L ; acide glutamique, 5 g/L ; extrait de levure, 1 g/L, $K_2HPO_4$, 1 g/L ; $MgSO_4$, 0,5 g/L ; KCl, 0,5 g/L ; $CuSO_4$, 1,6 mg/L ; $Fe_2(SO_4)_3$, 1,2 mg/L ; $MnSO_4$, 0,4 mg/L.

2.2 Solutions stocks

Pour assurer une reproductibilité de la composition du milieu, des solutions concentrées stériles ont été réalisées. Une solution de glucose 10X (200 g/L) a été stérilisée par autoclavage à 121°C durant 20 minutes. Une solution d'acide glutamique 4X (20 g/L) a été ajustée à pH 8 par une solution de KOH 5 M et a été stérilisée par filtration sur filtre de porosité 0,2 $\mu$m. Une solution d'extrait de levure 20X (20 g/L) a été stérilisée par autoclavage à 121°C durant 20 minutes. Une solution de minéraux n°1 40X ($K_2HPO_4$, 40 g/L ; $MgSO_4$, 20 g/L, KCl, 20 g/L) a été acidifiée par du $H_2SO_4$ concentré jusqu'à dissolution totale des sels et a été stérilisée par filtration sur filtre 0,2 $\mu$m. Une solution de sels minéraux n°2 40X ($CuSO_4$, 64 mg/L ; $Fe_2(SO_4)_3$, 48 mg/L ; $MnSO_4$, 16 mg/L) a été acidifiée par de l'acide sulfurique concentré jusqu'à dissolution totale des sels et a été stérilisée par filtration sur filtre de porosité 0,2 $\mu$m.

2.3 Réalisation d'un litre de milieu de Landy

Le matériel utilisé, hormis les pipettes, qui sont stériles et à usage unique, a préalablement été stérilisé par autoclavage à 121°C durant 20 minutes. 250 mL de la solution d'acide glutamique ont été prélevés stérilement et ont ensuite été versés dans une fiole d'Erlenmeyer. 100 mL de la solution de glucose y ont été ajoutés stérilement, puis 50 mL de la solution d'extrait de levure et enfin 25 mL de chacune des solutions de minéraux. Le pH a été ajusté à 7,0 à l'aide d'une solution de KOH 5M stérile. Le volume a été complété à 1 L avec de l'eau stérile.

2.4 Réalisation d'un milieu de Landy tamponné à 100 mM de MOPS

Un tampon MOPS 20X (2M) a été réalisé en dissolvant 20,93 g d'acide 3-[N-Morpholino]propanesulfonique (MOPS) (M1254, Sigma, St Louis, MO, U.S.A.) dans 50 mL d'eau. La solution a été stérilisée sur filtre de porosité 0,2 μm sous une hotte à flux laminaire. Pour réaliser 1 L de milieu de Landy tamponné à 100 mM par du MOPS, 50 mL de MOPS 20X ont été ajoutés au mélange réalisé à l'exemple 2.3.

**Exemple 3 : Culture de *B. subtilis* BBG125 en fioles d'Erlenmeyer**

3.1 Préparation d'un souchier

Un tube à vis contenant 5 mL de milieu E modifié (Le milieu E de Clark est modifié en diminuant la concentration en glucose de 40 à 20 g/L. La composition du milieu est la suivante : $KH_2PO_4$, 2,7 g/L ; $K_2HPO_4$, 18,9 g/L; Extrait de levure, 0,5 g/L; Glucose, 20 g/L; EDTA, 0,05 g/L; $MgSO_4$, 0,61 g/L; $MnSO_4$, 0,056 g/L; NaCl, 0,1 g/L; $CaCl_2$, 0,012 g/L; $ZnSO_4$, 0,018 g/L; $FeSO_4$, 0,018 g/L; $CuSO_4$, 0,002 g/L; $Na_2MoO_4$ 0,001 g/L; $H_3BO_3$, 0,001 g/L; $Na_2SO_3$, 0,001 g/L; $NiCl_2$, 0,0037 g/L; $NH_4NO_3$, 4 g/L; $MgSO_4$, 1 g/L. Le pH de la solution est ajusté à 6,5 par une solution d'HCl à 10%) a été inoculé par une colonie du souchier primaire de *B. subtilis* BBG125 et mis à incuber à 30°C pendant 48 h sous une agitation de 300 rotations par minute (rpm). Le tube a ensuite été homogénéisé au vortex. Un volume de 1,5 mL de la culture obtenue précédemment a été ajouté à 48,5 mL de milieu E modifié contenus dans une fiole d'Erlenmeyer de 500 mL. Le tout a été mis à incuber à 30°C pendant 12 à 24 h sous une agitation de 120 rpm. Cette première préculture P1 a été doublée.

La culture a ensuite été homogénéisée au vortex, la $DO_{600nm}$ a alors été mesurée par un spectrophotomètre (SECOMAN Prim (SECOMAN, Domont, France) jusqu'à ce que la souche *B. subtilis* BBG125 soit en début/milieu de phase exponentielle de croissance.

Une préculture P2 a été inoculée par 1,5 mL de la culture de la meilleure fiole de P1 et a été doublée. Les fioles d'Erlenmeyer de 500 mL contenaient, comme volume final, 50 mL de milieu E modifié et ont été incubées à 30°C sous une agitation de 120 rpm. La croissance a été arrêtée lorsque la $DO_{600nm}$ indiquait que la culture était en début/milieu de phase exponentielle de croissance ($1<DO_{600nm}<5$). La pureté et la qualité de P2 ont été contrôlées, par une observation au microscope et par ensemencement d'une gélose nutritive de Luria-Bertani (Tryptone, 10 g/L; Extrait de levure, 5 g/L; NaCl, 10 g/L ; pH 7,2. et d'une gélose de Mossel (Extrait de viande, 1 g/L; Peptone, 10 g/L; D-mannitol, 10 g/L; NaCl, 10 g/L; Rouge de phénol, 0,025 g/L; Agar, 12 g/L; Jaune d'oeuf, 10 mL/L; Polymyxine, 5 mL/L; pH 7,1)), plus spécifique des bacilles, complémentée en spectinomycine à 100 μg/mL. Les boîtes ont été mises à incuber à 30°C pendant 24 h.

Il est à noter que *B. subtilis* produit des colonies de formes irrégulières (les contours sont ondulés et peuvent présenter des filaments), de consistance crémeuse et dont le diamètre est compris entre 2 et 4 mm. Dans les vieilles cultures, les colonies prennent un aspect sec, rugueux et elles s'incrustent dans la gélose.

Pour finir, une fiole de 2 L contenant 200 mL de milieu E modifié défini ci-dessus a été inoculée à 5% avec la meilleure fiole de P2. Cette fiole a été incubée à 30°C sous une agitation de 120 rpm et la croissance a été arrêtée lorsque la $DO_{600nm}$ indiquait que la culture est en début/milieu de phase exponentielle de croissance ($1<DO_{600nm}<5$). La qualité et la pureté de la culture ont été contrôlées comme indiqué pour P2. La culture a été centrifugée à 2000 g pendant 10 min à 25°C. Les culots ont été lavés à l'eau physiologique stérile puis les suspensions ont été centrifugées à 2000 g pendant 10 min à 25°C. Les culots ont été repris dans un volume de milieu E sans antibiotique, de façon à obtenir une $DO_{600nm}$ finale de 25 par tube. La suspension a été répartie en cryotubes à raison de 0,9 mL de culture et 0,6 mL de glycérol. Les tubes ont été homogénéisés au vortex et conservés à -80°C. Le milieu E a été complémenté avec de la spectinomycine à 100 μg/mL.

3.2 Préparation d'un inoculum

L'inoculum a été préparé à partir du souchier contenant des cellules conservées à -80°C dans 40% de glycérol. Un tube contenant 5 mL de milieu E modifié défini ci-dessous a été ajusté à pH 7,0 avec une solution d'HCl 10% (v/v) a été inoculé par 0,5 mL de suspension bactérienne du souchier. L'ensemble a été mis à incuber à 30°C pendant 10 à

14 h sous une agitation de 300 rpm. Puis, le tube a été homogénéisé au vortex et la $DO_{600nm}$ a été mesurée. Une préculture P1 est alors réalisée dans un volume final de 50 mL de milieu E modifié à pH 7,0 contenu dans une fiole d'Erlenmeyer de 500 mL. Le tout a été mis à incuber à 30°C sous une agitation de 140 rpm, la préculture a été arrêtée lorsque la souche se situe en début/milieu de phase exponentielle de croissance ($1<DO_{600nm}<5$). Cette première préculture P1 a été doublée.

Une seconde préculture P2 a été réalisée de la même manière que la préculture P1, celle-ci a été inoculée à partir de la meilleure fiole de P1 et a été doublée. Le volume nécessaire pour démarrer les cultures en fioles a alors été centrifugé à 2000 g pendant 10 min à 25°C. Le culot a été remis en suspension dans 10 mL d'eau physiologique stérile. La suspension obtenue a été centrifugée à nouveau à 2000 g pendant 10 min. Le culot a enfin été repris dans 10 mL d'eau physiologique stérile. La suspension était alors prête pour l'inoculation.

3.3 Cultures en fioles d'Erlenmeyer

Les expériences ont duré au maximum 72 h et plusieurs échantillonnages ont été effectués sur ces cultures. La $DO_{600nm}$ initiale est comprise entre 0,1 et 0,4. Le volume des fioles d'Erlenmeyer était de 500 mL et le volume de milieu nutritif de 100 mL. On a réalisé sur les échantillons prélevés stérilement sous la hotte à flux laminaire les mesures suivantes : un contrôle de la pureté par isolement sur gélose nutritive et gélose Mossel + spectinomycine (100 µg/mL), une mesure de la densité optique à 600 nm, une mesure du pH, une mesure du poids sec et un prélèvement du surnageant de culture pour le dosage des lipopeptides par CLHP : 3 mL de culture sont centrifugés 10 min à 10000 g à 4°C et le surnageant de culture a été conservé à -20°C.

**Exemple 4 : Purification et analyse des lipopeptides**

4.1 Purification des lipopeptides

L'extraction des lipopeptides a été réalisée sur des cartouches de 1 g de gel C18 Maxi-clean (Grace Davison-Alltech, Deerfield, IL, U.S.A.). Une cartouche d'1 g d'ODS a été conditionnée avec du méthanol 100%, par 20 mL au premier passage puis 8 mL. La cartouche a ensuite été rincée par 8 mL d'eau milli-Q (Millipore). 1 mL de surnageant de culture dont le pH est égal à pH 6,5 +/-0,1, a alors été chargé sur la colonne. La cartouche a ensuite été lavée avec 8 mL d'eau milli-Q. Après séchage de la cartouche avec 20 mL d'air, les lipopeptides ont été élués par 4 mL de méthanol 100%. L'éluat a été porté à sec à l'aide d'un concentrateur sous vide. L'échantillon a par la suite été repris dans 200 µL de méthanol 100% à 4°C pour permettre le dosage en CLHP.

4.2. Analyses par chromatographie liquide haute performance (CLHP)

L'échantillon a été analysé grâce à un système complet de CLHP de la marque Waters (Online Degaser, 717 Autosampler, 660S Controller, 626 Pump, 2996 PhotoDiodeArray) (Waters SAS, Guyancourt, France) en employant une colonne C18 (5 µm, 250 x 2,5 mm, VYDAC 218 TP). Deux dosages ont été effectués.

Le premier dosage était celui des mycosubtilines : 10 µL d'échantillon purifié ont été injectés et confrontés à un standard d'iturine A à 500 mg/L (11774, Sigma-Aldrich, St Louis, MO, U.S.A.) avec un débit de 0,6 mL/min. L'élution a été réalisée en mode isocratique à l'aide d'un solvant eau/ acétonitrile/ acide trifluoroacétique 60/40/0,1 (v/v/v).

Le second dosage était celui des surfactines. 10 µL d'échantillon purifié ont été injectés et confrontés à un standard de surfactine à 500 mg/L (S3523, Sigma-Aldrich, St Louis, MO, U.S.A.) avec un débit de 0,6 mL/min. L'élution a été réalisée en mode isocratique à l'aide d'un solvant eau/ acétonitrile/ acide trifluoroacétique 20/80/0,1 (v/v/v).

Le temps de rétention et la dérivée seconde du spectre entre 200 et 400 nm de chaque pic (barrette de diodes, PDA 2996, Waters) ont été analysés automatiquement à l'aide du logiciel Millenium pour l'identification des molécules éluées.

4.3 Analyses par CLHP semi-préparative

L'échantillon a été préparé en appliquant le protocole de purification décrit à l'exemple 4.1 ci-dessus en employant des cartouches de gel C18 Maxi-clean (Grace Davison-Alltech, Deerfield, IL, U.S.A.) de 10 g. L'utilisation de cartouches de 10 g a permis de charger 10 mL de surnageant de culture au lieu de 1 mL précédemment. Tous les volumes ont été multipliés par un facteur 10, sauf les volumes de méthanol. Le volume minimum de méthanol utilisé pour le conditionnement de la cartouche puis l'élution des lipopeptides a été de 10 mL. L'échantillon a été chargé manuellement (100 µL) dans le système d'injection de la CLHP semi-préparative de marque Waters (660 Controller, 626 Pump, 486 Absorbance Detector). La colonne employée était une C18 (5 µm, 300 x 10 mm, ACE). L'élution a été réalisée à un débit de 3 mL/min selon le gradient présenté dans le tableau 2 suivant :

**Tableau 2 : Élution en fonction des concentrations respectives des tampons A et B**

| Temps (min) | Tampon A (%) | Tampon B (%) |
|---|---|---|
| 0 | 65 | 35 |
| 4 | 65 | 35 |
| 54 | 50 | 50 |
| 60 | 0 | 100 |
| 61 | 65 | 35 |
| 65 | 65 | 35 |

Les solvants utilisés étaient les suivants : le solvant A composé d'eau et d'acide trifluoroacétique, 99,9/ 0,1 (v/v) et le solvant B composé d'acétonitrile et d'acide trifluoroacétique, 99,9/ 0,1 (v/v).

4.4 Analyses par spectrométrie de masse MALDI-TOF

Les analyses par spectrométrie de masse MALDI-TOF (Bruker Ultraflex) ont été réalisées en fonction des besoins à partir : soit de surnageants de culture, soit d'échantillons purifiés sur cartouche ODS (Grace Davison-Alltech, Deerfield, IL, U.S.A.) ou soit d'échantillons purifiés sur cartouche ODS puis par CLHP semi-préparative. Un tampon TA a été préparé en réalisant un mélange $CH_3CN$/ eau/ acide trifluoroacétique, 33/67/0,1 (v/v/v). Un tampon CHCA est une solution saturée en acide alpha-cyano-4-hydroxycinnamique dans du tampon TA. Ce tampon a été préparé en récupérant le surnageant après centrifugation du mélange acide alpha-cyano-4-hydroxycinnamique / tampon TA. Les échantillons à analyser ont été préparés en mélangeant 1 $\mu$L de l'échantillon à 9 $\mu$L de tampon CHCA. La solution d'échantillon déposée pour l'analyse MALDI-TOF représentait un volume de 0,5 $\mu$L. Le séchage a été réalisé à l'air libre. La calibration des masses a été réalisée avec un mélange de peptides standards.

Les masses calculées des ions $[M+H]^+$, $[M+Na]^+$, $[M+K]^+$ des différents homologues de mycosubtilines et de surfactines obtenues sont précisées dans le tableau 3 ci-dessous :

**Tableau 3 : Masses calculées des ions [M+H]+, [M+Na+], [M+K+] des différents homologues de mycosubtilines et de surfactines**

| Lipopeptides | Masses | | |
|---|---|---|---|
| | $[M+H]^+$ | $[M+Na]^+$ | $[M+K]^+$ |
| **Surfactine $C_{13}$** | 1008,66 | 1030,64 | 1046,61 |
| **Surfactine $C_{14}$** | 1022,67 | 1044,66 | 1060,63 |
| **Surfactine $C_{15}$** | 1036,69 | 1058,67 | 1074,65 |
| **Mycosubtiline $C_{15}$** | 1057,57 | 1079,55 | 1095,52 |
| **Mycosubtiline $C_{16}$** | 1071,58 | 1093,56 | 1109,54 |
| **Mycosubtiline $C_{17}$** | 1085,60 | 1107,58 | 1123,55 |

L'analyse HPLC a révélé dix pics dont les masses moléculaires des molécules détectées sont présentés dans le tableau 4 ci-dessous :

**Tableau 4 : Masses des 10 pics détectés par l'analyse HPLC**

| Pic n° | Masse | | Pic n° | Masse |
|---|---|---|---|---|
| **1** | 1056 | | **6** | 1098 |
| **2** | 1084 | | **7** | 1098 |
| **3** | 1084 | | **8** | 1084 |
| **4** | 1070 | | **9** | 1084 |

(suite)

| Pic n° | Masse | | Pic n° | Masse |
|---|---|---|---|---|
| **5** | 1070 | | **10** | 1098 |

4.5 Analyses de la structure des nouvelles mycosubtilines par MS-MS

Afin de déterminer précisément la structure des différentes formes de mycosubtilines produites par la souche BBG125, une analyse des échantillons purifiés a été réalisée par spectrométrie de masse en tandem (MS-MS) avec une ionisation de type électrospray (Ion Trap Finnigan MAT LCQ) en mode infusion directe après ouverture du cycle peptidique au moyen d'un traitement au N-bromosuccinimide en concentration équivalente à la mycosubtiline dans une solution d'acide acétique à 70%.

Une première analyse a été réalisée sur la mycosubtiline *anteiso* C17 purifiée (pic 8). Le spectre obtenu (MS1) est compliqué par les 2 isotopes du Br. Le spectre MS2 est compliqué par la présence de fragments avec 1, 2 ou 3 groupements -NH3 en moins, dus à la présence des acides aminés Asn et Gln. Le spectre MS du produit de départ donne les pics 1085 (M+H)+, 1107 (M+Na)+ et 1123 (M+K)+ correspondant bien à la mycosubtiline C17.

Vue la présence des isotopes 79Br et de 81 Br en quantités assez semblables, on observe une distribution de pics autour de 1260 qui correspondent bien au dérivé attendu. Par exemple, le pic à 1257,4 correspond à [M+H]+ avec deux 79Br, le pic à 1259,4 correspond à [M+H]+ avec 79Br et 81 Br ou à [M+H]+ avec deux 13C et deux 79Br, etc...

L'ordre des masses croissantes obtenues à partir de la fragmentation de l'ion à 1257,4 est repris dans le tableau 5 ci-dessous :

**Tableau 5 : Masses croissantes obtenues à partir de la fragmentation de l'ion à 1257,4**

| m/z | Fragment d'ion | | m/z | Fragment d'ion |
|---|---|---|---|---|
| **365** | vN-NH3 | | **717,1** | y3 |
| **382,4** | vN | | **814,0** | y4-NH3 |
| **391,6** | b4-H2O-NH3 | | **831,1** | y4 |
| **432** | y2-NH3 | | **918** | y5 |
| **434,4** | SNv-H2O-NH3 | | **664** | y6-3NH3 |
| **452,3** | SNv-NH3 | | **981,1** | y6-2NH3 |
| **462,3** | NvN-2NH3 | | **998,1** | y6-NH3 |
| **496,4** | NvN | | **1015,1** | y6 |
| **506,2** | b5-2NH3 | | **1091,9** | y7-3NH3 |
| **531,3** | SNvN-H2O-2NH3 ou PSNv-H2O-NH3 | | **1109,1** | y7-2NH3 |
| **549,4** | SNvN-2NH3 ou PSNv-NH3 | | **1126,0** | y7-NH3 |
| **566,3** | PSNv ou SNvN-NH3 | | **1143** | y7 |
| **583,4** | SNvN | | **1240,0** | M-H2O |
| **700** | y3-NH3 | | - | - |

Dans le tableau ci-dessus, « *m/z* » signifie rapport masse sur charge.

4.6 Analyse MS-MS du pic à 1274,4 contenu dans le pic 6

Masse isotopique avant traitement : M=1098,6
Masse isotopique après traitement, avec 79Br2 : M=1270,4

Fragments y identiques à la mycosubtiline *anteiso* $C_{17}$.

Fragments b supérieurs de 14 par rapport aux fragments b de la mycosubtiline *anteiso* $C_{17}$.

Cela signifie que très probablement un des 2 premiers acides aminés de la séquence ouverte (N ou Q de NQPSNvNw) a été modifié.

<u>4.7 Analyse MS/MS du pic à 1274,4 contenu dans le pic 10</u>

Masse isotopique avant traitement : M=1098,6
Masse isotopique après traitement, avec 79Br2 : M=1270,4

Fragments y identiques à la mycosubtiline *anteiso* $C_{17}$ mais avec 14 en plus, dont le pic y6 intense à 1029 au lieu de 1015 pour tous les autres échantillons.
Fragments b inférieurs à b6 identiques à la mycosubtiline *anteiso* C17.
Fragments b supérieurs ou égaux à b6 identiques à la mycosubtiline mais avec 14 en plus.
Ces résultats nous montrent que la molécule serait de la mycosubtiline avec un acide gras en $C_{18}$ et non en $C_{17}$.
Sur la base de l'ordre d'élution des différents pics, nous en déduisons l'existence de 5 nouvelles formes de mycosubtiline : les formes Gln3 : *iso* $C_{16}$, nC16, *anteiso* $C_{17}$, *iso* $C_{17}$ et une forme en $C_{18}$. La correspondance des ces formes avec les dix pics dont les masses moléculaires des molécules détectées par l'analyse HPLC de l'exemple 4.5 est présentée dans le tableau 6 ci-dessous :

**Tableau 6 : Correspondance entre les masses des 10 pics détectés par l'analyse HPLC et les formes de mycosubtiline**

| Pic n° | Masse | Forme | | Pic n° | Masse | Forme |
|---|---|---|---|---|---|---|
| **1** | 1056 | $C_{15}$ | | **6** | 1098 | *anteiso*I$C_{17}$, |
| | | | | | | GLN |
| **2** | 1084 | *iso*$C_{16}$ GLN | | **7** | 1098 | *iso*$C_{17}$ GLN |
| **3** | 1084 | $_{n}C_{16}$ GLN | | **8** | 1084 | *anteiso*$C_{17}$ |
| **4** | 1070 | *iso*$C_{16}$ | | **9** | 1084 | *iso*$C_{17}$ |
| **5** | 1070 | *n*$C_{16}$ | | **10** | 1098 | $C_{18}$ |

La formule de cette nouvelle mycosubtiline $C_{18}$ est la suivante :

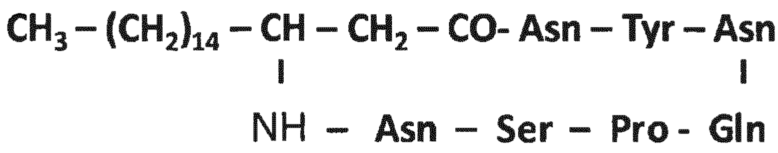

(I)

La formule de cette nouvelle mycosubtiline Gln3 $C_{17}$ est la suivante :

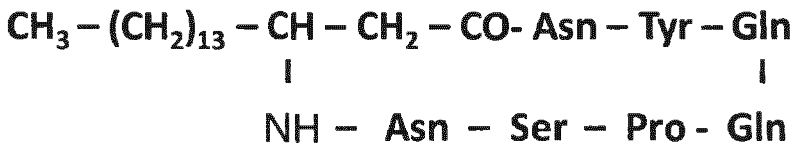

(II)

**Exemple 5 : Activité biologique de différentes isoformes de mycosubtiline (MS) vis-à-vis de divers microorganismes**

Des tests d'activités antifongiques ont été réalisés par dilutions successives de l'isoforme $C_{18}$ (MS $C_{18}$) en milieu liquide selon le protocole décrit dans Besson *et al.* (Besson et al. 1979. Antifungal activity upon Saccharomyces

cerevisiae of iturin A, mycosubtilin, bacillomycin L and of their derivatives; inhibition of this antifungal activity by lipid antagonists. J. Antibiot. (Tokyo) 32, 828-833 **[19]**). Des cultures en microplaques de 96 puits ont été réalisées dans un milieu riche : glucose, 40 g/L ; Peptone, 10 g/L ; Extrait de levure, 2 g/L ; pH=7,2. Un ensemencement de *Saccharomyces cerevisiae* a été réalisé à une $DO_{600nm}$ de 0,55 et la lecture d'absorbance s'est faite après 24 h et la concentration minimale inhibitrice (CMI) a ensuite été déterminée.

Cette expérience a également été réalisée avec les isoformes de mycosubtiline (MS) suivantes : MS *iso*-$C_{16}$, MS *n*-$C_{16}$, MS *anteiso*-$C_{17}$, MS *iso*-$C_{17}$. Elle a aussi été réalisée avec une composition de mycosubtilines (MS comp.) comprenant, en pourcentage par rapport au poids de la composition, 26% de MS *iso*-$C_{16}$, 1% MS Gln3 $C_{17}$, 2% MS *n*-$C_{16}$, 44% MS *anteiso*-$C_{17}$, 23% MS *iso*-$C_{17}$ et 1% MS $C_{18}$.

Cette expérience a également été réalisée, pour chacune des isoformes de mycosubtilines et composition précitées sur les microorganismes suivants : *Botrytis cinerea, Aspergillus niger, Sclerotinia sclerotium, Candida albicans.*

Les CMI obtenues pour chacune de ces expériences sont présentées dans le tableau 7 ci-dessous :

**Tableau 7 : CMI de différentes isoformes de mycosubtiline et d'une composition vis-à-vis de divers microorganismes**

| **Microorganismes** | **CMI (µM)** | | | | |
|---|---|---|---|---|---|
| | MS *iso*-$C_{16}$ | MS *n*-$C_{16}$ | MS *anteiso*-$C_{17}$ | MS *iso*-$C_{17}$ | MS comp. |
| *B. cinerea* | 32 | 16 | 8 | 16 | 8 |
| *A. niger* | 32 | 16 | 8 | 16 | 8 |
| *C. albicans* | > 32 | 8 | 32 | 16 | 8 |

Ces résultats montrent que la composition **MS comp.** présente des effets équivalents, voire supérieurs, aux mycosubtilines actuellement utilisées.

**Exemple 6: Mise en oeuvre d'un procédé intégré de production, d'extraction et de concentration des lipopeptides produits par *B. subtilis* sur contacteur membranaire air/liquide**

La description de cet exemple se réfère aux figures 1 à 5.

Dans cet exemple :

- les pompes **P1, P2, P3, P4, P5, P6, P7, P8, P9, P10, P11, P12, P13** et **P14** étaient des pompes péristaltiques Masterflex L/S compact drive model (Cole Parmer, Vernon Hills, IL, U.S.A.),
- la pompe **P11** était du type N820.3 FT.18 (KNF Neuberger Laboport, Freiburg, Allemagne),
- les vannes **V1, V4** et **V5,** étaient des vannes d'arrêt en PTFE, (W3250Y, Thermo Fisher Scientific, Roskilde, Allemagne),
- les vannes **V2, V3, V6** et **V7** étaient des vannes d'arrêt trois voies en PTFE (W3250Z, Thermo Fisher Scientific, Roskilde, Allemagne),
- les cuves **Cuve 2** et **Cuve 4** étaient des cuves Nalgene en polypropylène haute densité avec un volume utile de 4 L (2125-4000 Heavy Duty Bottles, Nalgene, Thermo Fisher Scientific, Roskilde, Allemagne) les cuves **Cuve 1, Cuve 3, Cuve 5, Cuve 6** et **Cuve 7** étaient des cuves Nalgene en polypropylène haute densité avec un volume utile de 10 ou 20 L (2250 Autoclavable Carboys, Nalgene, Thermo Fisher Scientific, Roskilde, Allemagne),
- les balances **B1, B2, B3, B4, B5, B6,** et **B7** étaient du type (CKW-55 ; Ohaus Corporation, Pine Brook, NJ, U.S.A.),
- la souche de *Bacillus subtilis* était la souche *B. subtilis* BBG125.

6.1 Conditions environnementales et sondes utilisées pour la culture

Sauf indication contraire, le pH a été régulé à une valeur de 7+/-0,1 grâce à l'ajout contrôlé respectivement par les pompes **P2** et **P3** de solutions de $H_3PO_4$ 0,66 M ou de NaOH 3 M stérilisées préalablement par autoclavage à 121°C durant 20 min.

Une électrode de pH a été calibrée avant autoclavage de la cuve en utilisant des solutions commerciales tamponnées à pH 4,0 et pH 7,0 et conservées à 4°C. Le processus a été conduit à 22 +/-0,1°C au moyen d'un échangeur thermique tubulaire de 1 $m^2$ Alpha Laval (104878, Alpha Laval Corporate AB, Lund, Suède). La concentration en oxygène dissous $pO_2$ a été mesurée grâce à une sonde à oxygène (Mettler Toledo, Viroflay, France). L'électrolyte de la sonde à oxygène a été renouvelé à chaque expérience. La sonde à oxygène a été calibrée après autoclavage de la cuve lorsque le milieu de culture atteignait la température et le pH de consigne de l'expérience. Le 0 % de $pO_2$ a été obtenu

en reliant le câble de la sonde à la masse et le 100 % $pO_2$ en saturant le milieu en air (1000 rpm et 1 vvm).

Le débit d'aération (Fe) a été fixé à 0,25 volume d'air par volume de liquide par minute (vvm), soit 0,75 L/min pour 3 L de milieu de Landy (exemple 2.1). L'air entrant était filtré à travers un filtre stérilisant de 0,2 $\mu$m.

Le logiciel utilisé pour la commande du processus et l'acquisition des données était AFS Biocommand (New Brunswick Scientific, Edison, NJ, U.S.A.). La pureté de la culture a été vérifiée après 48 h et en fin de culture. Des échantillons de culture de 10 mL ont régulièrement été prélevés et centrifugés, la densité optique et le poids sec ont été déterminés, le surnageant a été conservé avant analyse. Les gaz entrants et sortants ont été analysés afin d'obtenir des données sur la respiration du microorganisme. Un capteur paramagnétique a permis d'analyser la quantité d'oxygène et un capteur infrarouge celle du dioxyde de carbone (Xentra 4400 ; Servomex Compagny Inc., Sugar Land, TX, U.S.A.). L'analyseur s'intégrait à un dispositif multiplexé qui a permis une analyse séquentielle sur six voies, un séchage des gaz sur membrane Naflon (Permapur, Saint-Léonard, Québec) et une calibration automatique.

6.2 Le contacteur membranaire air/liquide

Le contacteur membranaire air/liquide **M1** utilisée dans cet exemple est fourni par GE-Healthcare référence CFP-6-D-45 (GE-Healthcare Europe GmbH, Munich, Allemagne). Il est constitué d'un module externe comprenant deux compartiments **C1** et **C2.** Dans le compartiment **C1,** circule un gaz stérile contenant de l'oxygène. Dans le compartiment **C2,** circule le milieu de culture contenant l'inoculum à un débit de 24 L/h/$m^2$ de membrane imposé par la pompe **P4.**

La membrane **M1** a une surface de 2,5 $m^2$ et est stérilisée avant utilisation par autoclavage à 121°C pendant 20 min (ce critère n'est pas exhaustif). La membrane est constituée d'un ensemble de fibres creuses de polyéthersulfone ayant une porosité de 0,65 $\mu$m.

6.3 Dispositif permettant une culture en continu de *Bacillus subtilis* : Couplage d'un système d'extraction/concentration des biosurfactants au contacteur membranaire air/liquide

*6.3.1. Couplage d'un système d'extraction/concentration des lipopeptides au contacteur membranaire air/liquide*

Le dispositif employé pour la culture en continu de *B. subtilis* comporte un contacteur membranaire air/liquide **M1** en fibres creuses dans lequel *B. subtilis* BBG125 a été cultivée en milieu de Landy. *B. subtilis* BBG125 immobilisé à la surface de la membrane M1 dégrade ce substrat en excrétant des biosurfactants. Le dispositif comporte également des moyens pour alimenter et soutirer en continu dans le dispositif de production comprenant la membrane **M1** un débit déterminé dudit substrat.

La membrane **M1** apporte dans un environnement stérile, l'oxygène nécessaire à la croissance du microorganisme dans le milieu de culture, grâce à la diffusion de l'oxygène à travers ses pores.

Une pompe péristaltique **P1** permet d'alimenter en continu la membrane **M1,** à un débit **F1,** en milieu de Landy frais stocké dans une **Cuve 1,** de même une pompe péristaltique **P5** permet de soutirer en continu le milieu de culture du dispositif de production comprenant la membrane **M1** décrit ci-dessus à un débit **F2.**

L'inoculum et les conditions de fermentation restent équivalents à ceux décrits précédemment. Afin de maintenir l'environnement stérile, l'ensemble des constituants du dispositif ainsi que les différentes membranes utilisées ont été stérilisés à 121°C pendant 20 min.

Une caractéristique de ce dispositif tient dans le fait que l'on peut recycler ou non dans le dispositif de production comprenant la membrane **M1** la totalité des cellules de microorganisme après les avoir débarrassées des résidus de matières organiques et des biosurfactants, ce qui permet d'obtenir un taux élevé de croissance des microorganismes dans le dispositif de production.

Par ailleurs ce dispositif permet la diffusion de l'oxygène dans le milieu de culture sans formation de bulles ou de mousse.

*6.3.2. Extraction des lipopeptides et le recyclage des cellules par microfiltration*

Le dispositif décrit précédemment est donc caractérisé par le fait qu'il comporte des moyens de microfiltration et d'ultrafiltration tangentielle, situés en sortie du dispositif de production qui séparent le liquide en plusieurs fractions.

Une première étape de microfiltration a été réalisée sur une membrane **M2** en fibres creuses de polyéthersulfone de taille de pores 0,2 $\mu$m (GE Healthcare) d'une surface de 0,4 $m^2$, dans laquelle le milieu de culture contenant les cellules circule, grâce à la pompe volumétrique **P4** décrite ci-dessus, dans le compartiment **C3** de la membrane **M2** appelé retentat. Sous l'effet d'une pompe volumétrique **P5** le milieu passe par filtration tangentielle à travers les pores de la membrane **M2** vers le compartiment **C4** de la membrane **M2.** Par ce biais, le milieu de culture contenant les biosurfactants est débarrassé des cellules et est extrait puis recueilli dans une **Cuve 2** agitée par des pales entraînées par un moteur **B** ou par agitation magnétique (Agitateur magnétique, W10512, Thermo Fisher Scientific, Roskilde,

Allemagne) à une vitesse de 160 rpm.

Le milieu de culture du dispositif de production comprenant la membrane **M1** est prélevé en continu vers la **Cuve 2.** Pour compenser ce soutirage et garder un volume constant dans ma membrane **M1,** la pompe **P1** alimente le bioréacteur en milieu neuf contenu dans la **Cuve 1.**

Par ailleurs, la **Cuve 2** et la **Cuve 3** sont placées respectivement sur des balances **B2** et **B3.** Ce sont ces dernières qui contrôlent le débit de la pompe péristaltique **P1** (CKW-55 ; Ohaus Corporation, Pine Brook, U.S.A.), en permettant de maintenir un volume constant à l'intérieur de la membrane **M1** et de ce fait d'obtenir F1 = F2. Dans chacune des expériences réalisées, le taux de dilution est changé après le passage d'au moins quatre volumes contacteur membranaire air/liquide comprenant la membrane **M1.**

Les débits des pompes **P1** et **P5** sont égaux et réglés pour obtenir dans le dispositif comprenant la membrane **M1** un taux de dilution de 0,1 $h^{-1}$, c'est-à-dire un débit horaire égal à 0,1 fois le volume de la phase aqueuse contenue dans le dispositif de production comprenant la membrane **M1.**

Une variante de ce procédé a également été réalisée. Elle réside dans le fait de recycler ou non les cellules à l'intérieur de la membrane **M1.** Il s'agit alors d'un mode continu ouvert présenté en pointillé sur les figures 3 et 4. Dans le cas d'un non-recyclage par le jeu des vannes **V6, V7** et **V8** le milieu de culture peut être pompé par la pompe **P13** depuis la membrane **M1** et collecté dans la **Cuve 7.** Une vanne de purge permet d'éliminer le concentrat cellulaire par intermittence. Dans ce cas, l'étape de microfiltration par la membrane **M2** est réalisée directement sur le milieu contenu dans la **Cuve 7** et les cellules sont alors concentrées dans la **Cuve 7** et non plus dans le dispositif de production comprenant la membrane **M1.**

*6.3.3. Concentration des lipopeptides par ultrafiltration*

Le filtrat contenu dans la **Cuve 2** est enfin entraîné, grâce à une pompe **P6,** dans le compartiment **C5** d'un système d'ultrafiltration tangentielle en inox (Sartocon 2 plus, 17546---202, Sartorius, Göttingen, Allemagne), comprenant une membrane d'ultrafiltration **M3** de seuil de coupure de 10 kDa en cellulose régénérée (Hydrosart Ultrafilter, 3021443930E-BSW, Sartorius, Göttingen, Allemagne). Au-dessus de la concentration micellaire critique, les biosurfactants sont concentrés dans le compartiment **C5,** appelé retentat et retournent ainsi dans la **Cuve 2.** Le milieu de culture est extrait, sous le contrôle d'une pompe **P7** à un débit équivalent à celui de la pompe **P5,** vers un compartiment **C6,** appelé ultrafiltrat, et recueilli dans une **Cuve 3.** Le débit sera fonction du débit imposé par la pompe **P7** et régulé grâce aux informations collectées par les différentes balances **B2, B3** et **B4.**

<u>6.4 Purification des lipopeptides</u>

*6.4.1. Purification par ultrafiltration/diafiltration*

La purification des lipopeptides présenté dans cet exemple repose sur l'enchaînement d'étapes d'ultrafiltration à travers un système d'ultrafiltration tangentielle en inox (Sartocon 2 plus, 17546---202, Sartorius, Göttingen, Allemagne), comprenant une membrane d'ultrafiltration **M4** de seuil de coupure de 10 kDa en cellulose régénérée (Hydrosart Ultrafilter, 3021443930E-BSW, Sartorius, Göttingen, Allemagne). Cette étape vise à éliminer du bouillon de culture une grande partie des substances résiduelles telles que le glucose, le glutamate et les différents métabolites primaires. La formation de micelles et de complexes micellaires par la mycosubtiline, quand elle se trouve au-dessus de sa CMC, permet de la retenir et donc de la concentrer dans le retentat, grâce à l'utilisation de la membrane d'ultrafiltration **M4.**

Cette étape se déroule à 25°C et sous une pression de 0,5 bar. Cette étape de purification s'effectue sur les lipopeptides concentrés dans la **Cuve 2.** Après ouverture de la vanne **V1,** la pompe **P8** entraîne le concentrat vers la **Cuve 4** (Nalgene en polypropylène haute densité avec un volume utile de 4 L (2125-4000 Heavy Duty Bottles, Nalgene, Thermo Fisher Scientific, Roskilde, Allemagne)) agitée par des pales entraînées par un moteur **C** ou par agitation magnétique (Agitateur magnétique, W10512, Thermo Fisher Scientific, Roskilde, Allemagne) identique au moteur **B**.

Les étapes qui suivent sont réalisées séquentiellement :

- Une ultrafiltration : Le concentrat est transféré dans la **Cuve 4,** sous le contrôle d'une pompe **P8** péristaltique Masterflex L/S compact drive model (Cole Parmer, Vernon Hills, IL, USA), puis purifié sur la membrane **M4,** sous le contrôle d'une pompe **P9** péristaltique Masterflex L/S compact drive model (Cole Parmer, Vernon Hills, IL, USA) et recueilli dans la **Cuve 4.** La pompe **P10** péristaltique Masterflex L/S compact drive model (Cole Parmer, Vernon Hills, IL, USA) permet de faire passer le reste des constituants du milieu vers le compartiment **C8** et la **Cuve 5** (Nalgene en polypropylène haute densité avec un volume utile de 10 ou 20 L (2250 Autoclavable Carboys, Nalgene, Thermo Fisher Scientific, Roskilde, Allemagne)). Ce processus est mené à bien jusqu'à ce que le volume contenu dans la **Cuve 4** soit réduit à 10% du volume initialement dans la **Cuve 4.**
- Une diafiltration : Cette étape, permet de diluer le bouillon de culture afin de faciliter le passage des substances

résiduelles à travers la membrane d'ultrafiltration **M4.** De l'eau est alors ajoutée dans la **Cuve 4** grâce à l'ouverture de la vanne **V2** jusqu'à ce que le volume de la **Cuve 4** retrouve son niveau d'origine. Il s'ensuit une étape d'ultrafiltration comme décrit ci-dessus. Cette étape de diafiltration est réalisée 4 fois de suite.

- Une ultrafiltration en présence de méthanol (MeOH) : A la suite des étapes de diafiltration du MeOH est ajouté depuis la **Cuve 6** (Nalgene en polypropylène haute densité avec un volume utile de 10 ou 20 L (2250 Autoclavable Carboys, Nalgene, Thermo Fisher Scientific, Roskilde, Allemagne) vers la **Cuve 4** via la pompe **P12** péristaltique Masterflex L/S compact drive model (Cole Parmer, Vernon Hills, IL, USA) et la vanne **V2.** Cet ajout de MeOH est contrôlé par les balances **B4, B5** et **B6** pour que la solution présente à ce moment dans la **Cuve 4** contienne 70% de MeOH (v/v). Il s'ensuit une étape d'ultrafiltration comme décrit ci-dessus. Cette étape va permettre la destruction des micelles et le passage des monomères de mycosubtiline à travers les pores de la membrane **M4.**

Après filtration, une solution constituée de mycosubtiline et de méthanol à 70% est recueilli du côté ultrafiltrat, mais cette fois-ci l'ultrafiltrat est recueilli dans la cuve d'un évaporateur **(Evapo)** Rotavapor VV2000 (Heidolph Instruments GmbH & Co, Schwabach, Allemagne) grâce au jeu des vannes **V3** et **V4.**

A la fin de chaque étape, des échantillons sont prélevés du côté filtrat et du côté retentat. Ainsi le bilan de la purification peut être établi. Celui-ci permet de déterminer les pertes en mycosubtiline provoquées par les étapes d'ultrafiltration et de diafiltration, en faisant le rapport de la quantité de mycosubtiline concentrée obtenue après ultrafiltration et de la quantité de mycosubtiline initialement présente dans la **Cuve 4.** Le rendement de ces étapes est supérieur à 70%.

*6.4.2. Concentration des lipopeptides par évaporation*

L'évaporateur **(Evapo)** permet de concentrer les mycosubtilines en retirant tout le méthanol et une partie de l'eau. Cette évaporation s'effectue sous une pression résiduelle de 50 mbar imposée par la pompe à vide **P11,** type N820.3 FT.18 (KNF Neuberger Laboport, Freiburg, Allemagne) et à 50°C. Durant cette étape, le méthanol est évaporé et ses vapeurs sont condensées grâce au condenseur et celui-ci est recyclé dans la **Cuve 6.**

*6.4.3. Lyophilisation des lipopeptides*

Une étape facultative de lyophilisation a été ajoutée à ce procédé pour améliorer la conservation du produit. La lyophilisation des lipopeptides est effectuée directement à partir de la solution concentrée **X** issue de l'évaporation et récupérée par la vanne **V5.** Celle-ci est d'abord congelée à -20°C, puis lyophilisée grâce à un lyophilisateur Heto Power Dry PL 9000 (Jouan Nordic, Allerod, Danemark), selon les étapes suivantes : 1 h à -30°C ; 5 h à -10°C ; 5 h à 0°C ; 5 h à -20°C ; 5 h à 35°C. La lyophilisation a été effectuée sous une pression résiduelle de 15 mbar.

**Exemple 7 : Lavage des membranes et récupération des lipopeptides**

Compte tenu de l'affinité des lipopeptides pour les interfaces, le protocole de lavage des membranes a été étudié et optimisé. Il tient compte de la nature des membranes et il est en accord avec les récents travaux publiés sur ce sujet (Chen, Chen and Juang, 2007. Separation of surfactin from fermentation broths by acid precipitation and two-stage dead-end ultrafiltration processes. J. Membr. Sci. 299, 114-121 **[20];** Chen, Chen, and Juang, 2008. Flux decline and membrane cleaning in cross-flow ultrafiltration of treated fermentation broths for surfactin recovery. Sep. Purif. Technol. 62, 47-55 **[21]**). Ces lavages ne dénaturent ni les lipopeptides ni les membranes. L'utilisation de solvants est proscrite bien que certains, comme le méthanol, soient très efficaces pour décrocher les lipopeptides. Des tests de sensibilité au pH ont permis de déterminer qu'un pH=10 est la limite de dégradabilité des lipopeptides. Les lavages sont effectués dans les conditions d'agitation de la fermentation. Le protocole se déroule en sept étapes de lavage à base d'eau.

- Deux lavages ont été réalisés avec 3 L d'eau distillée à 30°C pendant 30 min. Ils ont permis de décrocher la biomasse faiblement immobilisée.
- Le deuxième lavage à l'eau distillée à 30°C a permis de réaliser une mesure du coefficient de transfert d'oxygène.
- Deux lavages ont été réalisés avec 3 L de NaOH 0,1 M à 50°C pendant 1 h. Ils ont permis de décrocher la biomasse fortement immobilisée et de désorber la majorité des lipopeptides.
- La membrane a alors été régénérée avec une solution de NaOH 0,5 M à 50°C pendant 1 h puis avec une solution de NaOCl 100 ppm à 50°C pendant 1 h, puis a été nettoyée à l'eau distillée à 25°C jusqu'à atteindre la neutralité dans la membrane.

L'ensemble du procédé précité a également été réalisé en doublant chaque membrane, afin qu'elle puisse être lavée et régénérée séquentiellement. Cela a permis de réaliser le procédé sans discontinuer. Afin de réaliser ce procédé

alternatif, des cuves (non représentées) contenant la solution de soude à 0,1 et 0,5 M ont été ajoutées au dispositif.

**Exemple 8 : Quantification des biosurfactants produits**

Dans cet exemple, plusieurs essais ont été réalisés pour produire des biosurfactants par *B. subtilis* par fermentation du glucose à une concentration de 20 g/L, dans un dispositif de production contenant 3 L de milieu de culture et une surface totale de contacteur membranaire air/liquide de 2,5 $m^2$, conforme au contacteur membranaire air/liquide décrite dans l'exemple 6. Chacune des expériences a été répétée deux fois. Seule la moyenne de ces doublons est présentée ci-après. La déviation standard est comprise entre 5 et 15%.

Les conditions d'expérimentation étaient réglées de la manière suivante :

- le pH était maintenu à 7,
- le débit d'air dans le contacteur membranaire air/liquide était de 1 vvm,
- le volume de milieu de culture était de 3 L circulant à l'intérieur des fibres de ladite membrane à une vitesse de 0,021 m/s,
- la pression de l'ensemble du système était la pression atmosphérique, sauf au niveau de l'entrée d'air, celle-ci peut être légèrement au-dessus de la pression atmosphérique à 0,4 bar,
- les conditions d'oxygénation du milieu de culture ont été fixées avec un coefficient volumétrique de transfert d'oxygène proche de 40 $h^{-1}$.

L'analyse chromatographique par HPLC a permis la quantification des substrats et des biosurfactants dans les différentes cuves.

L'analyse de l'oxygène consommé a permis de déterminer la quantité de cellules immobilisées sur la membrane.

La formule suivante a été utilisée pour déterminer la biomasse immobilisée sur la membrane au cours du temps en (g $m^{-2}$) en considérant que les cellules libres et immobilisées ont une vitesse spécifique de consommation d'oxygène différente, l'oxygène étant plus accessible pour les cellules immobilisées sur la membrane que pour celles en suspension :

**biomasse immobilisée sur la membrane à un temps donné**

$$\textbf{en (g m}^{-2}\textbf{) = (OUR-(X* OUR}_{spe\ cl}\textbf{))*V/( OUR}_{spe\ ci}\textbf{ *a)}$$

dans laquelle :

OUR = Vitesse de consommation d'oxygène

$OUR_{spe\ cl}$= Vitesse spécifique de consommation d'oxygène des cellules libres

$OUR_{spe\ ci}$= Vitesse spécifique de consommation d'oxygène des cellules immobilisées

V = Volume réactionnel

X = Concentration en biomasse libre

a = surface de la membrane

Les étapes de lavage de membranes décrites dans ces exemples ont été réalisées selon le procédé décrit dans l'exemple 7.

8.1 Production de mycosubtiline par *B. subtilis* BBG125, mode batch

Dans cet exemple, la souche *B. subtilis* BBG125 a été cultivée en batch à 30°C pendant 48 h dans un contacteur membranaire air/liquide **M1.**

*8.1.1 Analyse de la biomasse produite*

Dans ce procédé deux types de biomasse ont été observés, l'une en suspension libre dans le milieu de culture et l'autre immobilisée sur le contacteur membranaire air/liquide.

Les cellules libres ont été cultivées de façon exponentielle à 0,2 $h^{-1}$ de taux de croissance spécifique jusqu'à la 18ème heure. La biomasse libre a atteint un maximum de 2,6 g $L^{-1}$ après une journée de la culture, puis elle est demeurée constante jusqu'à la fin de la culture.

L'analyse des gaz a révélé la présence d'une biomasse immobilisée sur la membrane. La croissance de cette biomasse s'est déroulée durant le premier jour de culture pour atteindre 1,2 g $m^{-2}$. Une vitesse de consommation du glucose de 0,61 g $L^{-1}$ $h^{-1}$ a été mesurée lors du premier jour de culture, puis celle-ci a diminué en raison de l'appauvrissement de ce dernier dans le milieu..

*8.1.2 Analyse de la mycosubtiline produite*

La production de mycosubtiline a atteint 10 mg $L^{-1}$ après deux jours de culture. 45 mg ont été désorbés lors du lavage de la membrane résultant en une production totale de 25 mg $L^{-1}$ et une productivité moyenne de 0,5 mg $L^{-1}$ $h^{-1}$. Après purification par les étapes de microfiltration, ultrafiltration/diafiltration et séchage, un mélange de mycosubtiline en poudre contenant les nouvelles formes de mycosubtiline a été obtenu. La pureté de ce mélange était de plus de 94%.

<u>8.2 Production, extraction et purification en continu de mycosubtiline par *B. subtilis* BBG125 avec cellules totalement recyclées</u>

Dans cet exemple, la souche *B. subtilis* BBG125 (déposée le 10 mars 2011 sous le numéro CNCM I-4451 à la Collection Nationale de Cultures de Microorganismes (CNCM) de l'Institut Pasteur (Paris, France)), capable de produire uniquement de la mycosubtiline de manière constitutive, a été cultivée en continu à 22°C pendant 72 h dans un contacteur membranaire air/liquide **M1.**

En plus du contacteur membranaire air/liquide qui permet la croissance et l'immobilisation des cellules, le procédé présenté dans cet exemple se caractérise aussi par la présence de :

- dispositifs pour alimenter et pour soutirer en continu dans ledit réacteur un débit déterminé dudit substrat, conformes à ceux décrits dans l'exemple 6,
- une membrane de microfiltration **M2** de surface 0,45 $m^2$ et de taille de pores de 0,2 μm, conforme à celle décrite dans l'exemple 6,
- trois cuves : d'alimentation **(Cuve 1)**, de concentration **(Cuve 2)** et de déchet **(Cuve 3),** conformes à celles décrites dans l'exemple 6, et
- d'une membrane d'ultrafiltration **M3** de surface 0,1 $m^2$ et de seuil de coupure de 10 kDa, conforme à celle décrite dans l'exemple 6.

De plus, les débits d'alimentation et de soutirage des pompes **P1** et **P5** conformes à celles décrites dans cet exemple était égaux et réglés pour obtenir un taux de dilution de l'ordre de 0,1 $h^{-1}$, c'est-à-dire un débit horaire égal à 0,1 fois le volume de la phase aqueuse contenue dans le dispositif de production.

Une caractéristique de ce procédé, conforme à la présente invention, réside dans le fait que l'on recycle la totalité des cellules de microorganismes dans le dispositif de production après les avoir débarrassées des résidus de matières organiques et des biosurfactants, ce qui permet d'obtenir un taux élevé de croissance des microorganismes sur la membrane. La culture continue était précédée par 20 h de culture batch.

*8.2.1 Analyse de la biomasse*

Pendant les 40 premières heures de la culture continue à 0,1 $h^{-1}$ de taux de dilution, la biomasse libre n'a cessé d'augmenter dans le bouillon jusqu'à 7,2 g $L^{-1}$. Ensuite, sa concentration est restée constante, ce qui a révélé sûrement la présence d'un inhibiteur. En revanche l'analyse des gaz a révélé une immobilisation importante des cellules sur la membrane qui atteint 3,1 g $m^{-2}$ après 3 jours de culture. Au cours des 2 premiers jours de culture, la concentration de glucose a diminué avec une vitesse de consommation de glucose de 1,5 g $L^{-1}$ $h^{-1}$.

*8.2.2 Analyse de la surfactine produite*

La mycosubtiline a été ainsi extraite à travers la membrane de microfiltration et a bien été concentrée par la membrane d'ultrafiltration de 10 kDa ; son accumulation dans la cuve intermédiaire a été observée. La productivité de

mycosubtiline a augmenté au cours des 40 premières heures de la culture et a atteint un maximum de 1,5 mg $L^{-1}$ $h^{-1}$. Après cette expérience, le lavage des membranes permet de récupérer 84 mg de mycosubtiline. À la fin de cette expérience, la culture en continu a permis la production de 895 mg de mycosubtiline en solution, soit une concentration moyenne produite de 48 mg de mycosubtiline produite par litre de milieu consommé. Les étapes de l'ultrafiltration/diafiltration ont permis l'obtention de surfactine en solution avec une pureté de 90%. Ce procédé continu montre une productivité 3 fois supérieure à celle obtenue avec le mode batch décris à l'exemple 8.1

**Listes des références**

**[1]** Ongena, M. and Jacques, P. 2008. Bacillus lipopeptides: versatile weapons for plant disease biocontrol. Trends Microbiol. 16, 115-125.
**[2]** CZ20011620
**[3]** DE102005050123
**[4]** FR2578552
**[5]** Guez, J.S. et al., 2007. Setting up and modelling of overflowing fed-batch cultures of Bacillus subtilis for the production and continuous removal of lipopeptides, J. Biotechnol., 131, 67-75.
**[6]** Davis, D. A., Lynch, H. C. and Varley, J., 1999. The production of Surfactin in batch culture by Bacillus subtilis ATCC 21332 is strongly influenced by the conditions of nitrogen metabolism. Enzyme Microb. Technol. 25, 322-329.
**[7]** WO0226961
**[8]** EP1320595
**[9]** Landy M. et al. 1948. Bacillomycin ; an antibiotic from Bacillus subtilis active against pathogenic fungi. Proc. Soc. Exp. Biol. Med. 67, 539-541.
**[10]** Guez, J.S. et al., 2008. Respiration activity monitoring system (RAMOS), an efficient tool to study the influence of the oxygen transfer rate on the synthesis of lipopeptide by Bacillus subtilis. J. Biotechnol. 134, 121-126.
**[11]** Remize, P.J. et Cabassud, C. 2003. Un nouveau réacteur d'oxydation sans bulles : le contacteur à membrane G/L. Récents progrès en génie des procédés. Intégration des membranes dans les procédés 2. Lavoisier Tec et Doc.
**[12]** Duitman, E.H. et al., 1999. The mycosubtilin synthetase of Bacillus subtilis ATCC 6633: a multifunctional hybrid between a peptide synthetase, an amino transferase, and a fatty acid synthase. Proc. Natl. Acad. Sci. U.S.A., 96, 13294-13299.
**[13]** Leclère, V. et al., 2005. Mycosubtilin overproduction by Bacillus subtilis BBG100 enhances the organism's antagonistic and biocontrol activities. Appl. Environ. Microbiol. 71, 4577-4584.
**[14]** Sambrook, J. and Russell, D.W. 2001. Molecular cloning: a laboratory manual, 3rd ed., Cold Spring Harbor Laboratory, Cold Spring Harbor, New York.
**[15]** Dennis, J.J. and Zylstra, G.J. 1998. Plasposons: modular self-cloning minitransposon derivatives for rapid genetic analysis of gram-negative bacterial genomes. Appl. Environ. Microbiol. 64, 2710-2715.
**[16]** Herrero, M., de Lorenzo, V., and Timmis, K. N. 1990. Transposon vectors containing non-antibiotic resistance selection markers for cloning and stable chromosomal insertion of foreign genes in gram-negative bacteria, J. Bacteriol. 172, 6557-6567.
**[17]** Bertani, G. 2004. Lysogeny at mid-twentieth century: P1, P2, and other experimental systems. J. Bacteriol. 186, 595-600.
**[18]** Dubnau, D.A. 1982. Genetic transformation of Bacillus subtilis p148-178. In D.A. Dubnau (Ed) The molecular biology of the Bacilli, vol I. Bacillus subtilis. Academic Press, Inc. New York.
**[19]** Besson, F. et al. 1979. Antifungal activity upon Saccharomyces cerevisiae of iturin A, mycosubtilin, bacillomycin L and of their derivatives; inhibition of this antifungal activity by lipid antagonists. J. Antibiot. (Tokyo) 32, 828-833.
**[20]** Chen, H.L., Chen, Y.S., and Juang, R.S. 2007. Separation of surfactin from fermentation broths by acid precipitation and two-stage dead-end ultrafiltration processes. J. Membr. Sci. 299, 114-121.
**[21]** Chen, H.L., Chen, Y.S., and Juang, R.S. 2008. Flux decline and membrane cleaning in cross-flow ultrafiltration of treated fermentation broths for surfactin recovery. Sep. Purif. Technol. 62, 47-55.
**[22]** Coutte, F., et al.,. 2010, Effect of pps disruption and constitutive expression of srfA on surfactin productivity, spreading and antagonistic properties of Bacillus subtilis 168 derivatives. J. Appl. Microbiol. 109, 480-491


## Revendications

1. Mycosubtiline dont la chaine d'acide gras comporte 17 carbones et possédant une glutamine à la place de l'asparagine en position 3 dans son cycle peptidique (mycosubtiline Gln3 C17) et représentée par la formule (II) ci-dessous :

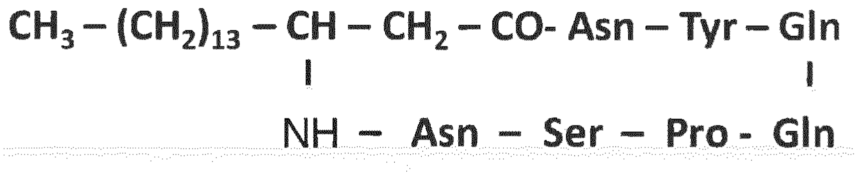

(II)

2. Composition comprenant au moins une mycosubtiline Gln3 C17 selon la revendication 1.

3. Composition selon la revendication 2, comprenant en outre au moins une mycosubtiline choisie dans le groupe comprenant une mycosubtiline *iso*-C16, une mycosubtiline n-C16, une mycosubtiline *anteiso*-C17, une mycosubtiline *iso*-C17, une mycosubtiline C18 et leurs mélanges.

4. Composition selon la revendication 2 ou 3 comprenant entre 1 et 60% de mycosubtiline *iso*-16, entre 1 et 20% de mycosubtiline Gln3 C17, entre 1 et 10% mycosubtiline n-C16, entre 20 et 95% de mycosubtiline *anteiso*-C17, entre 5 et 30% de mycosubtiline *iso*-C17 et entre 1 et 5% de mycosubtiline C18.

5. Mycosubtiline selon la revendication 1 , ou composition selon l'une quelconque des revendications 2 à 4, pour utilisation comme agent antifongique.

## Patentansprüche

1. Mycosubtilin, dessen Fettsäurekette 17 Kohlenstoffe enthält und das an der dritten Stelle seines Peptidzyklus ein Glutamin anstelle von Asparagin aufweist (Mycosubtilin Gin3 C17), und das durch die nachstehende Formel (II) dargestellt wird:

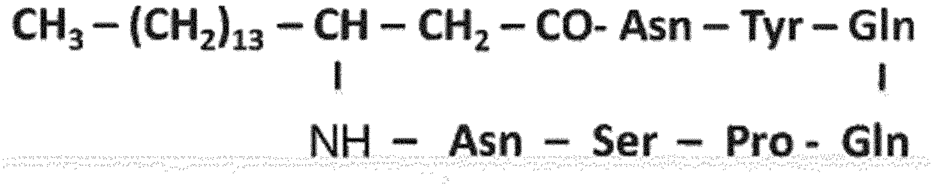

(II)

**2.** Zusammensetzung, die mindestens ein Mycosubtilin Gln3 C17 gemäß Anspruch 1 enthält.

**3.** Zusammensetzung nach Anspruch 2, welche des Weiteren mindestens ein Mycosubtilin enthält, das aus der Gruppe gewählt wurde, die ein Mycosubtilin *iso*-C16, ein Mycosubtilin n-C16, ein Mycosubtilin *anteiso*-C17, ein Mycosubtilin *iso*-C17, ein Mycosubtilin C18 und ihre Mischungen umfasst.

**4.** Zusammensetzung nach Anspruch 2 oder 3, welche zwischen 1 und 60% Mycosubtilin *iso*-16, zwischen 1 und 20 % Mycosubtilin Gln3 C17, zwischen 1 und 10% Mycosubtilin n-C16, zwischen 20 und 95% Mycosubtilin *anteiso*-C17, zwischen 5 und 30% Mycosubtilin *iso*-C17 und zwischen 1 und 5% Mycosubtilin C18 enthält.

**5.** Mycosubtilin nach Anspruch 1, oder Zusammensetzung nach einem der Ansprüche 2 bis 4, für die Verwendung als Antipilzwirkstoff.

## Claims

**1.** Mycosubtilin, the fatty acid chain of which contains 17 carbon atoms and having glutamine instead of asparagine in position 3 in the peptide ring (Gln3 C17 mycosubtilin) thereof and represented by the formula (II) below:

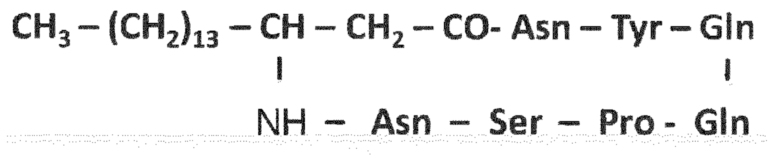

(II)

**2.** A composition comprising at least one Gln3 C17 mycosubtilin according to claim 1.

**3.** A composition according to claim 2, further comprising at least one mycosubtilin selected from the group comprising an *iso*-C16 mycosubtilin, a n-C16 mycosubtilin, an *anteiso*-C17 mycosubtilin, an *iso*-C17 mycosubtilin, a C18 mycosubtilin and the mixtures thereof.

**4.** A composition according to claim 2 or 3, comprising from 1 to 60% of *iso*-16 mycosubtilin, from 1 to 20% of Gln3 C17 mycosubtilin, from 1 to 10% of n-C16 mycosubtilin, from 20 to 95% of *anteiso*-C17 mycosubtilin, from 5 to 30% of *iso*-C17 mycosubtilin and from 1 to 5% of C18 mycosubtilin.

**5.** Mycosubtilin according to claim 1, or a composition according to any one of claims 2 to 4, for use as an antifungal agent.

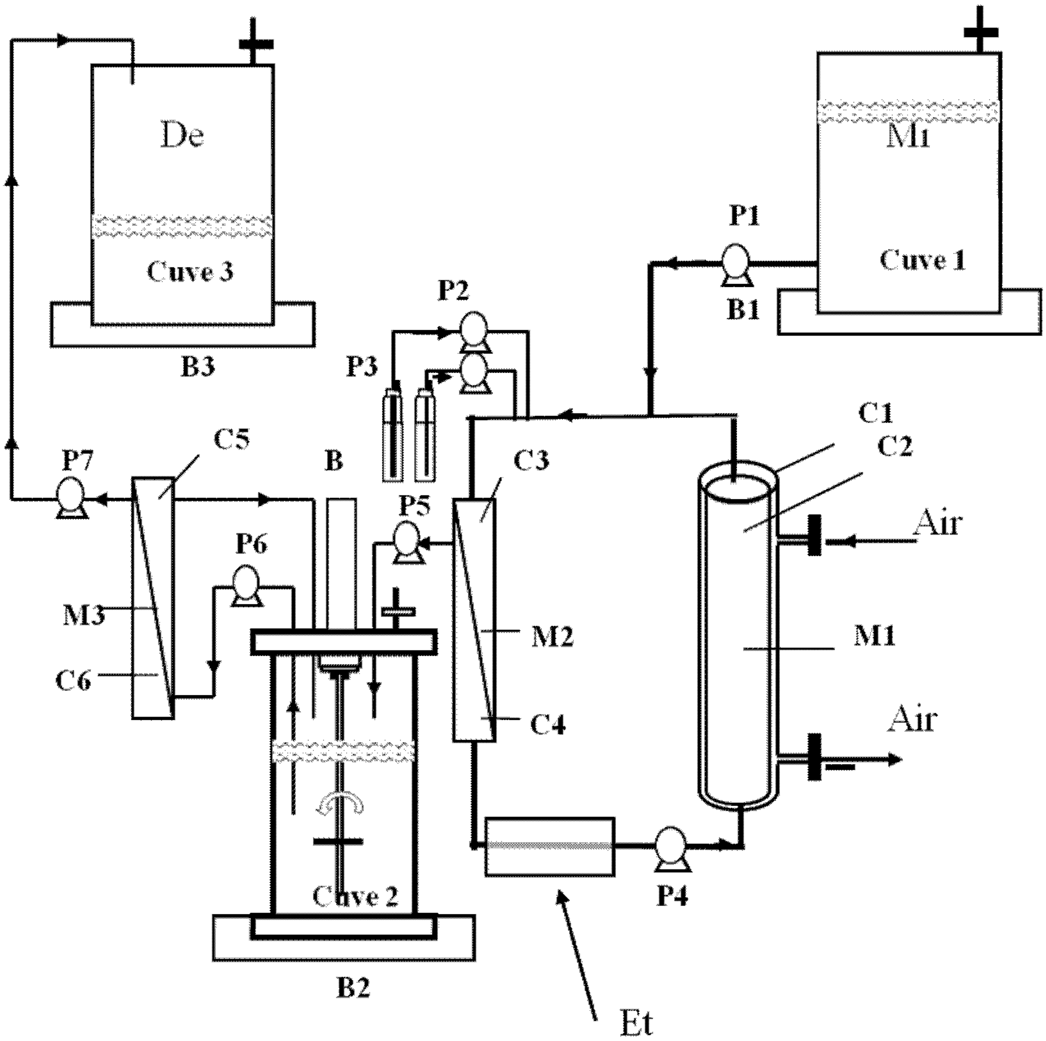
De
Cuve 3
B3
M1
Cuve 1
P1
B1
P2
P3
B
C5
P7
P5
C3
C1
C2
Air
P6
M3
M2
M1
C6
C4
Air
Cuve 2
P4
B2
Et

FIGURE 1

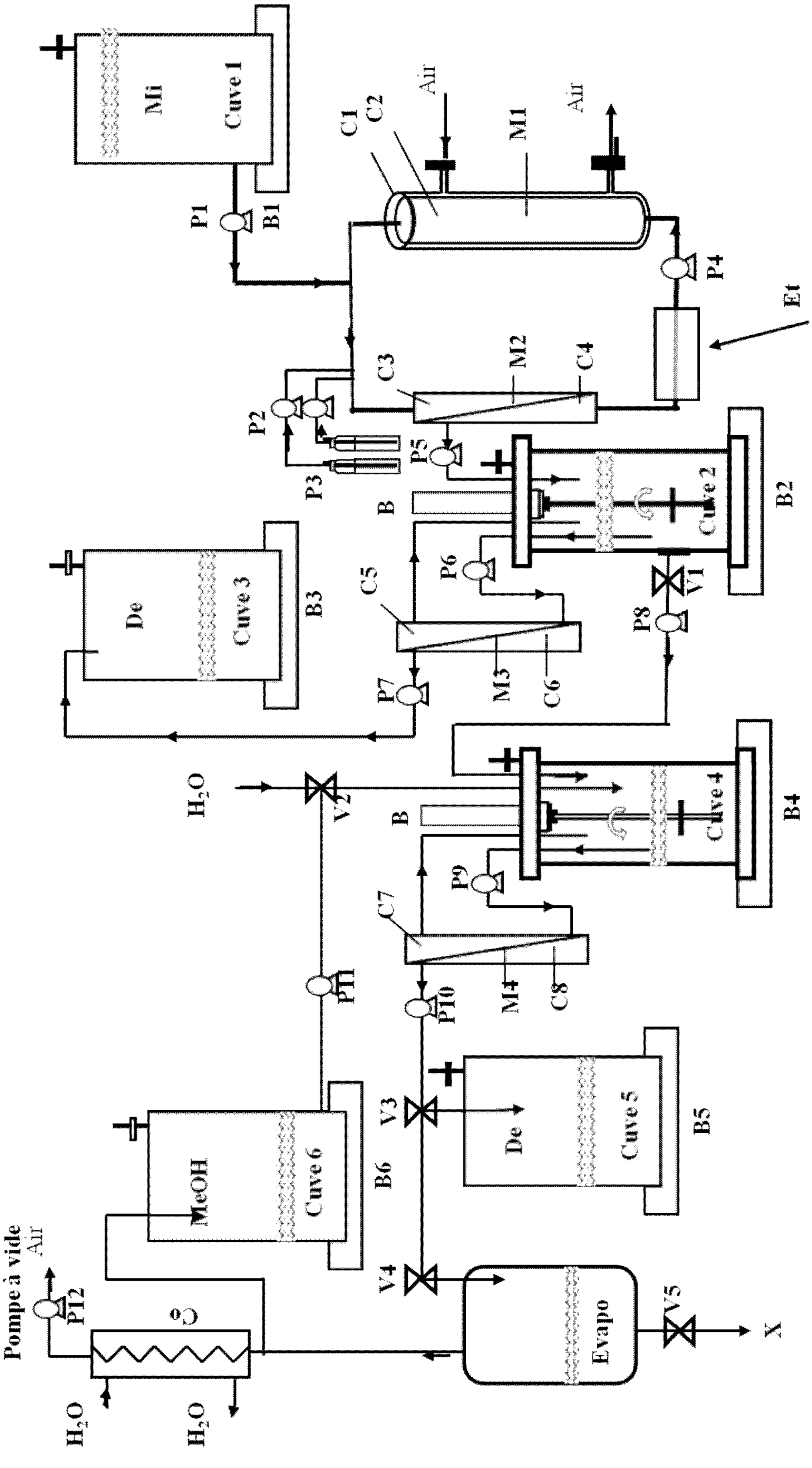
Mi
Cuve 1
B1
P1
Air
C1
C2
M1
Air
P4
Et
C3
M2
C4
P2
P3
P5
B
Cuve 2
B2
P6
V1
P8
De
Cuve 3
B3
C5
M3
C6
P7
H2O
V2
B
Cuve 4
B4
P9
C7
M4
C8
P10
P11
MeOH
Cuve 6
B6
V3
De
Cuve 5
B5
V4
Evapo
V5
X
Pompe à vide
Air
P12
Ce
H2O
H2O

FIGURE 2

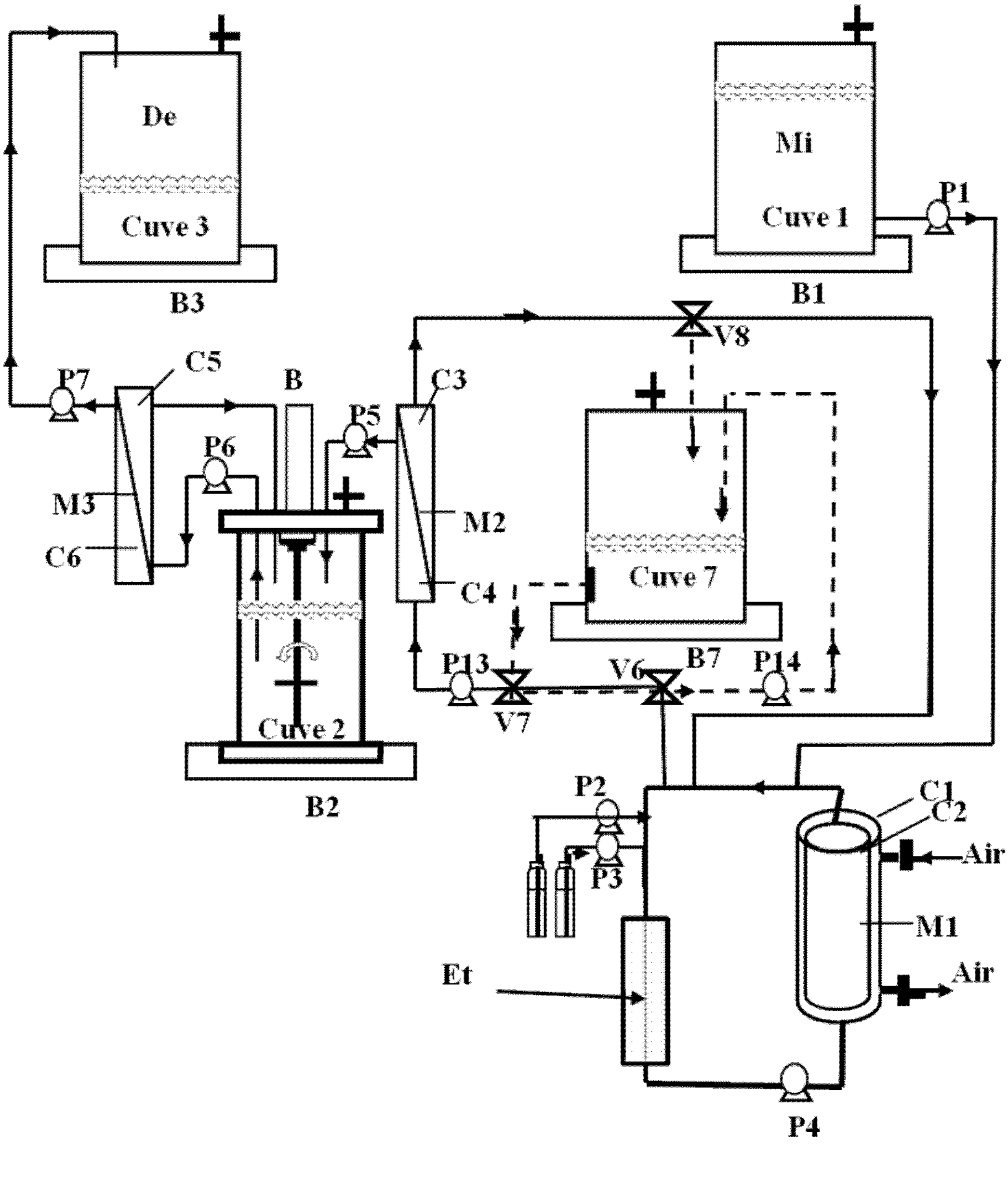
De
Cuve 3
B3
Mi
Cuve 1
B1
P1
V8
C3
C5
P7
B
P5
P6
M3
C6
M2
C4
Cuve 7
B7
P13
V6
P14
V7
Cuve 2
B2
P2
P3
C1
C2
Air
M1
Air
Et
P4

FIGURE 3

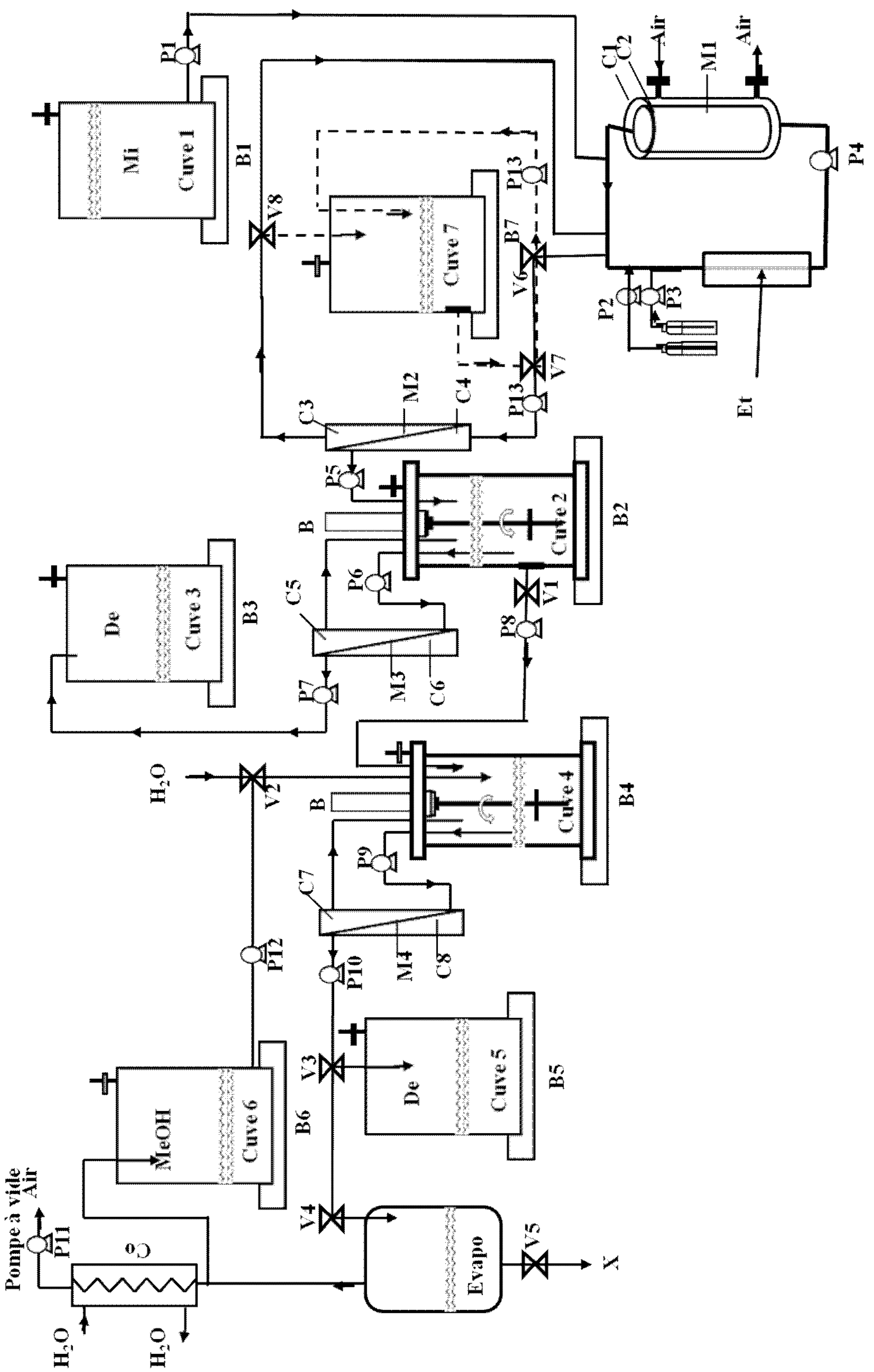
Mi
Cuve 1
B1
P1
Cuve 7
B7
V8
P13
V6
V7
C1
C2
Air
M1
P4
P2
P3
Et
C3
M2
C4
P5
B
Cuve 2
B2
P6
V1
De
Cuve 3
B3
C5
M3
C6
P7
P8
$H_2O$
V2
Cuve 4
B4
P9
C7
M4
C8
P10
P12
MeOH
Cuve 6
B6
De
Cuve 5
B5
V3
V4
Evapo
V5
X
Pompe à vide
P11
Co

FIGURE 4

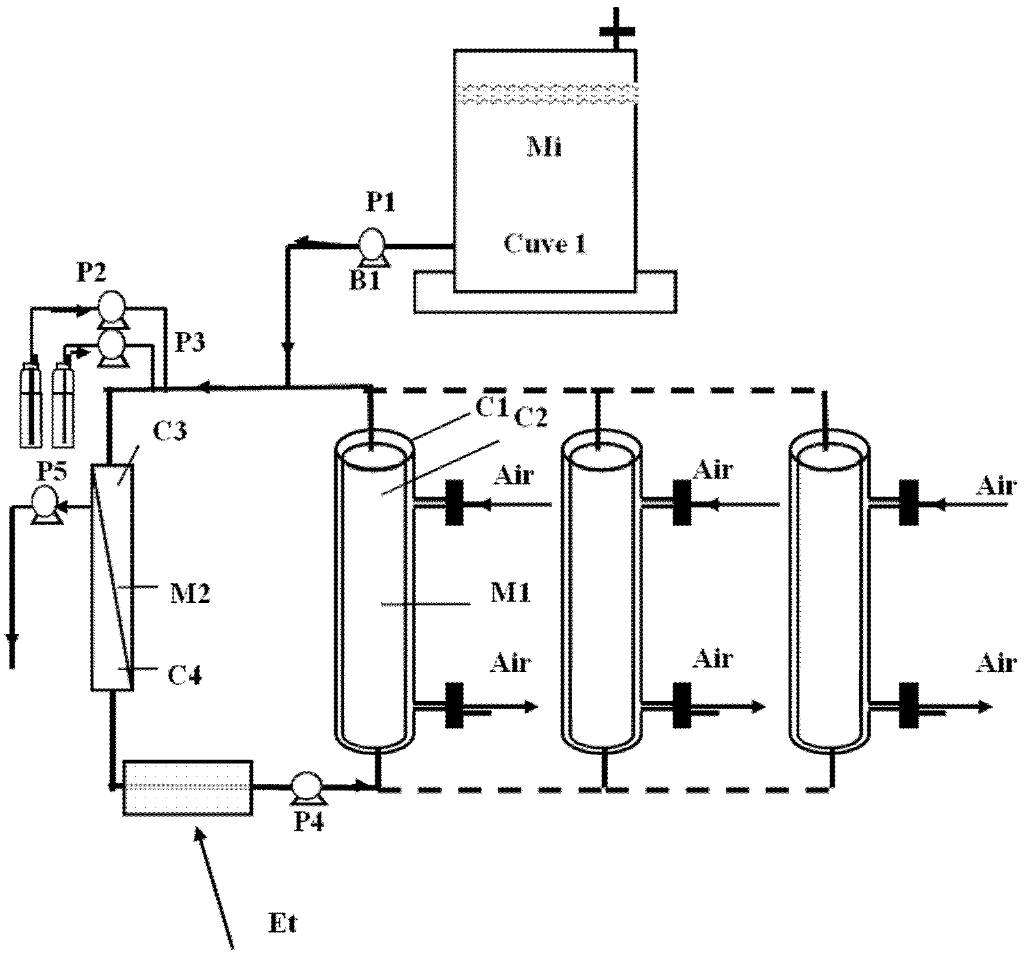
Mi
Cuve 1
P1
B1
P2
P3
C1
C2
C3
P5
Air
Air
Air
M2
M1
Air
Air
Air
C4
P4
Et

FIGURE 5

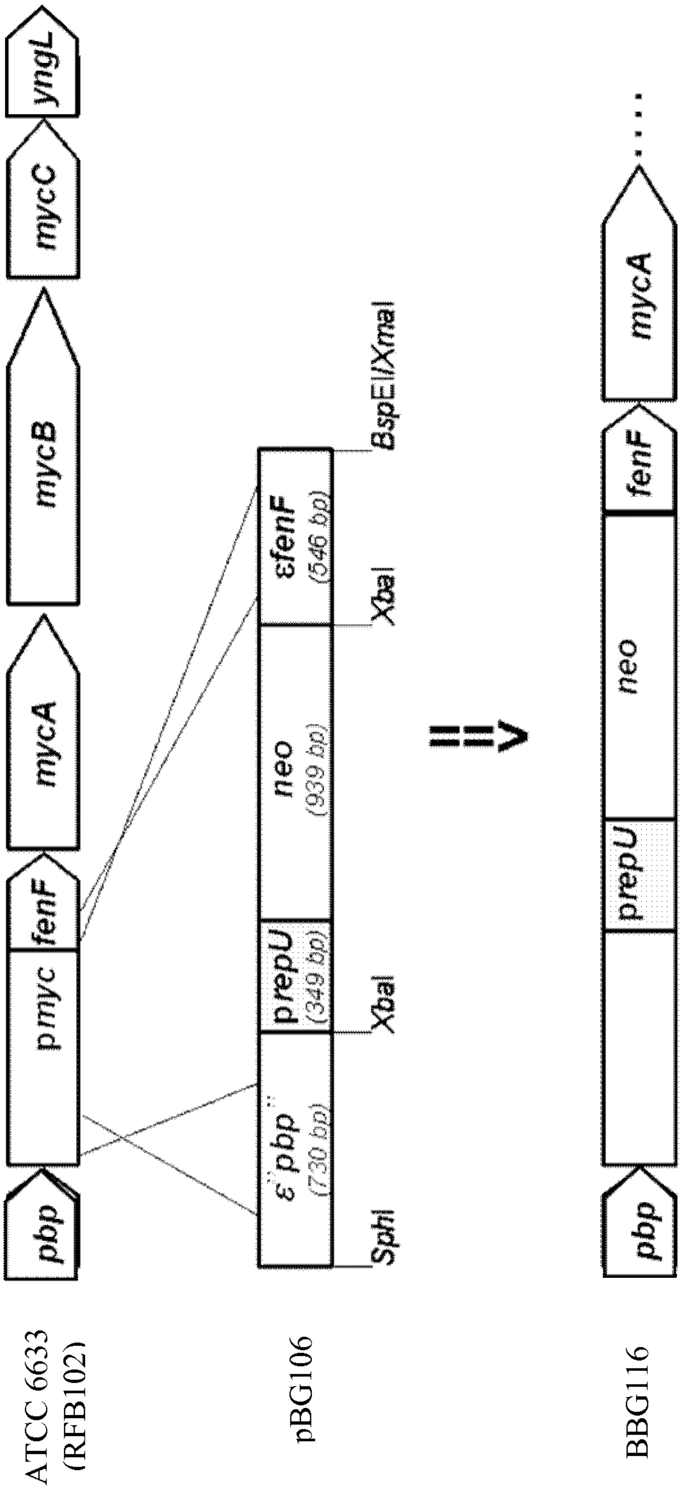

ATCC 6633
(RFB102)
pbp
pmyc
fenF
mycA
mycB
mycC
yngL
pBG106
ε''pbp''
(730 bp)
prepU
(349 bp)
neo
(939 bp)
εfenF
(546 bp)
SphI
XbaI
XbaI
BspEI/XmaI
BBG116
pbp
prepU
neo
fenF
mycA


# FIGURE 6

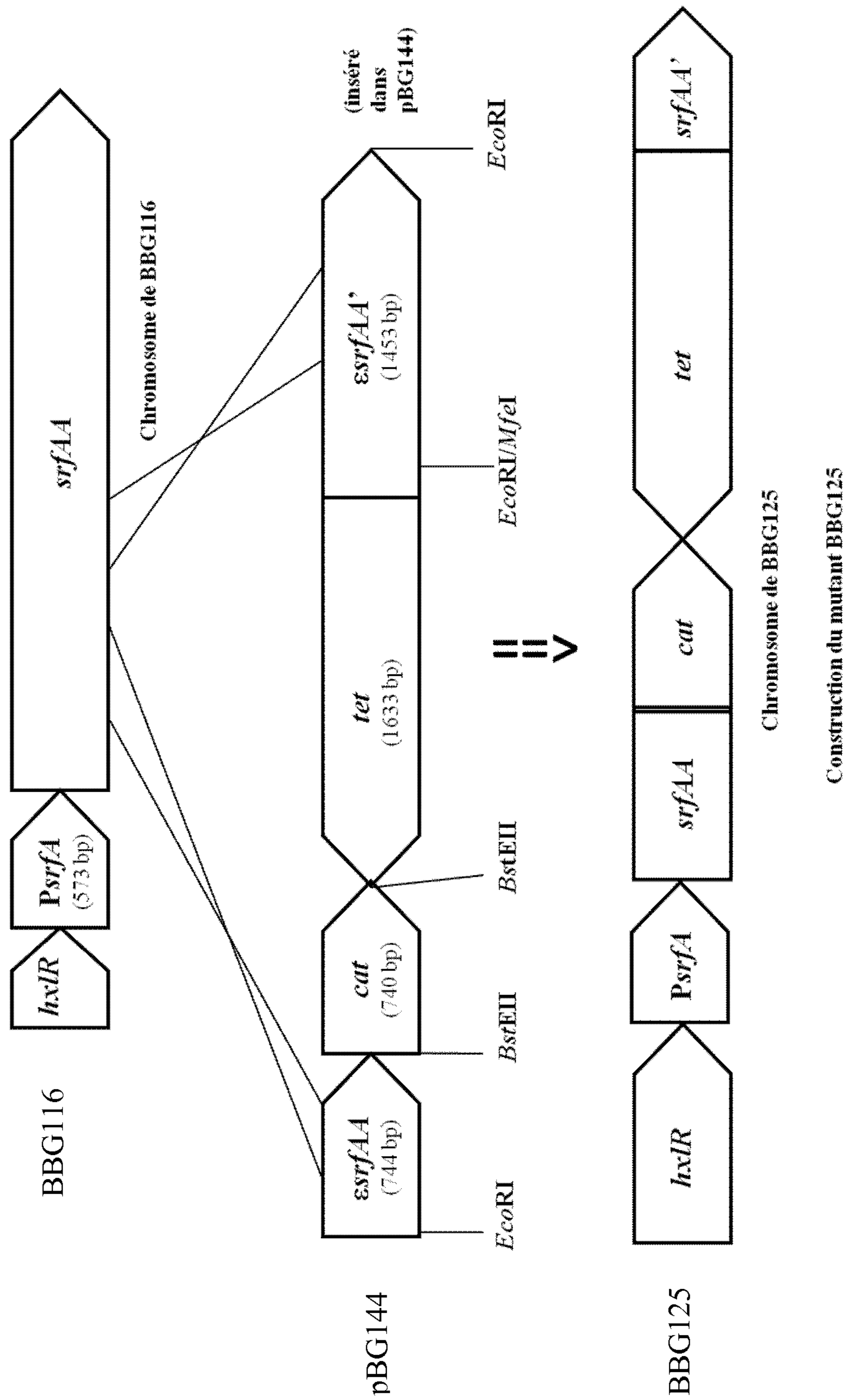
BBG116
hxlR
PsrfA
(573 bp)
srfAA
Chromosome de BBG116
pBG144
EcoRI
εsrfAA
(744 bp)
BstEII
cat
(740 bp)
BstEII
tet
(1633 bp)
EcoRI/MfeI
εsrfAA'
(1453 bp)
EcoRI
(inséré dans pBG144)
==>
BBG125
hxlR
PsrfA
srfAA
cat
tet
srfAA'
Chromosome de BBG125
Construction du mutant BBG125

FIGURE 7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- CZ 20011620
- DE 102005050123
- FR 2578552
- WO 0226961 A
- EP 1320595 A
- FR 1158922


### Littérature non-brevet citée dans la description


- **ONGENA ; JACQUES.** Bacillus lipopeptides: versatile weapons for plant disease biocontrol. *Trends Microbiol.,* 2008, vol. 16, 115-125
- **GUEZ et al.** Setting up and modelling of overflowing fed-batch cultures of Bacillus subtilis for the production and continuous removal of lipopeptides. *J. Biotechnol.,* 2007, vol. 131, 67-75
- **DAVIS ; LYNCH ; VARLEY.** The production of surfactin in batch culture by Bacillus subtilis ATCC 21332 is strongly influenced by the conditions of nitrogen metabolism. *Enzyme Microb. Technol.,* 1999, vol. 25, 322-329
- **LANDY et al.** Bacillomycin; an antibiotic from Bacillus subtilis active against pathogenic fungi. *Proc. Soc. Exp. Biol. Med.,* 1948, vol. 67, 539-541
- **GUEZ et al.** Respiration activity monitoring system (RAMOS), an efficient tool to study the influence of the oxygen transfer rate on the synthesis of lipopeptide by Bacillus subtilis. *J. Biotechnol.,* 2008, vol. 134, 121-126
- **REMIZE ; CABASSUD.** Un nouveau réacteur d'oxydation sans bulles : le contacteur à membrane G/L. Récents progrès en génie des procédés. *Intégration des membranes dans les procédés 2. Lavoisier Tec et Doc.,* 2003
- **DUITMAN et al.** The mycosubtilin synthetase of Bacillus subtilis ATCC 6633: a multifunctional hybrid between a peptide synthetase, an amino transferase, and a fatty acid synthas. *Proc. Natl. Acad. Sci. U.S.A.,* 1999, vol. 96, 13294-13299
- **LECLÈRE et al.** Mycosubtilin overproduction by Bacillus subtilis BBG100 enhances the organism's antagonistic and biocontrol activities. *Appl. Environ. Microbiol.,* 2005, vol. 71, 4577-4584
- **SAMBROOK ; RUSSELL.** Molecular cloning: a laboratory manual. Cold Spring Harbor Laboratory, 2001
- **DENNIS ; ZYLSTRA.** Plasposons: modular self-cloning minitransposon derivatives for rapid genetic analysis of gram-negative bacterial genomes. *Appl. Environ. Microbiol.,* 1998, vol. 64, 2710-2715
- **HERRERO ; DE LORENZO ; TIMMIS.** Transposon vectors containing non-antibiotic resistance selection markers for cloning and stable chromosomal insertion of foreign genes in gram-negative bacteria. *J. Bacteriol.,* 1990, vol. 172, 6557-67
- **BERTANI.** *Lysogeny at mid-twentieth century: P1, P2,* 2004
- *J. Bacteriol.,* vol. 186, 595-600
- Genetic transformation of Bacillus subtilis. **DUBNAU.** The molecular biology of the Bacilli, vol I. Bacillus subtilis. Academic Press, Inc, 1982, vol. I, 148-178
- **DUBNAU.** *Genetic transformation of Bacillus subtilis,* 1982, 148-178
- The molecular biology of the Bacilli. Bacillus subtilis. Academic Press, Inc, vol. I
- **BESSON et al.** Antifungal activity upon Saccharomyces cerevisiae of iturin A, mycosubtilin, bacillomycin L and of their derivatives; inhibition of this antifungal activity by lipid antagonists. *J. Antibiot. (Tokyo),* 1979, vol. 32, 828-833
- **CHEN ; CHEN ; JUANG.** Separation of surfactin from fermentation broths by acid precipitation and two-stage dead-end ultrafiltration processes. *J. Membr. Sci.,* 2007, vol. 299, 114-121
- **CHEN ; CHEN ; JUANG.** Flux decline and membrane cleaning in cross-flow ultrafiltration of treated fermentation broths for surfactin recovery. *Sep. Purif. Technol.,* 2008, vol. 62, 47-55
- **ONGENA, M. ; JACQUES, P.** Bacillus lipopeptides: versatile weapons for plant disease biocontrol. *Trends Microbiol.,* 2008, vol. 16, 115-125
- **GUEZ, J.S. et al.** Setting up and modelling of overflowing fed-batch cultures of Bacillus subtilis for the production and continuous removal of lipopeptides. *J. Biotechnol.,* 2007, vol. 131, 67-75
- **DAVIS, D. A. ; LYNCH, H. C. ; VARLEY, J.** The production of Surfactin in batch culture by Bacillus subtilis ATCC 21332 is strongly influenced by the conditions of nitrogen metabolism. *Enzyme Microb. Technol.,* 1999, vol. 25, 322-329

- **LANDY M. et al.** Bacillomycin ; an antibiotic from Bacillus subtilis active against pathogenic fungi. *Proc. Soc. Exp. Biol. Med.,* 1948, vol. 67, 539-541
- **GUEZ, J.S. et al.** Respiration activity monitoring system (RAMOS), an efficient tool to study the influence of the oxygen transfer rate on the synthesis of lipopeptide by Bacillus subtilis. *J. Biotechnol.,* 2008, vol. 134, 121-126
- **REMIZE, P.J. ; CABASSUD, C.** Un nouveau réacteur d'oxydation sans bulles : le contacteur à membrane G/L. Récents progrès en génie des procédés. *Intégration des membranes dans les procédés 2. Lavoisier Tec et Doc.,* 2003
- **DUITMAN, E.H. et al.** The mycosubtilin synthetase of Bacillus subtilis ATCC 6633: a multifunctional hybrid between a peptide synthetase, an amino transferase, and a fatty acid synthase. *Proc. Natl. Acad. Sci. U.S.A.,* 1999, vol. 96, 13294-13299
- **LECLÈRE, V. et al.** Mycosubtilin overproduction by Bacillus subtilis BBG100 enhances the organism's antagonistic and biocontrol activities. *Appl. Environ. Microbiol.,* 2005, vol. 71, 4577-4584
- **SAMBROOK, J. ; RUSSELL, D.W.** Molecular cloning: a laboratory manual. Cold Spring Harbor Laboratory, 2001
- **DENNIS, J.J. ; ZYLSTRA, G.J.** Plasposons: modular self-cloning minitransposon derivatives for rapid genetic analysis of gram-negative bacterial genomes. *Appl. Environ. Microbiol.,* 1998, vol. 64, 2710-2715
- **HERRERO, M. ; DE LORENZO, V. ; TIMMIS, K. N.** Transposon vectors containing non-antibiotic resistance selection markers for cloning and stable chromosomal insertion of foreign genes in gram-negative bacteria. *J. Bacteriol.,* 1990, vol. 172, 6557-6567
- **BERTANI, G.** Lysogeny at mid-twentieth century: P1, P2, and other experimental systems. *J. Bacteriol.,* 2004, vol. 186, 595-600
- Genetic transformation of Bacillus subtilis. **DUBNAU, D.A.** The molecular biology of the Bacilli, vol I. Bacillus subtilis. Academic Press, Inc, 1982, vol. I, 148-178
- **BESSON, F. et al.** Antifungal activity upon Saccharomyces cerevisiae of iturin A, mycosubtilin, bacillomycin L and of their derivatives; inhibition of this antifungal activity by lipid antagonists. *J. Antibiot. (Tokyo),* 1979, vol. 32, 828-833
- **CHEN, H.L. ; CHEN, Y.S. ; JUANG, R.S.** Separation of surfactin from fermentation broths by acid precipitation and two-stage dead-end ultrafiltration processes. *J. Membr. Sci.,* 2007, vol. 299, 114-121
- **CHEN, H.L. ; CHEN, Y.S. ; JUANG, R.S.** Flux decline and membrane cleaning in cross-flow ultrafiltration of treated fermentation broths for surfactin recovery. *Sep. Purif. Technol.,* 2008, vol. 62, 47-55
- **COUTTE, F. et al.** Effect of pps disruption and constitutive expression of srfA on surfactin productivity, spreading and antagonistic properties of Bacillus subtilis 168 derivatives. *J. Appl. Microbiol.,* 2010, vol. 109, 480-491

SEQUENCE **LISTING**

<110> Universite de Lille 1

<120> Biosurfactants de Bacilus sp., composition les comprenant, procédé d'obtention et application

<130> BIP206917PC00

<150> FR 11/58922
<151> 2011-10-03

<160> 14

<170> PatentIn version 3.5

<210> 1
<211> 21
<212> DNA
<213> Artificial Sequence

<220>
<223> amorce PBP-FO2

<400> 1
aataacggac atgccgaagtg 21

<210> 2
<211> 21
<212> DNA
<213> Artificial Sequence

<220>
<223> amorce FENF-REV2

<400> 2
aataggccga ccaagacgtt c 21

<210> 3
<211> 22
<212> DNA
<213> Artificial Sequence

<220>
<223> amorce SRF-FO

<400> 3
acaggaatat gctcaatcga ag 22

<210> 4
<211> 21
<212> DNA
<213> Artificial Sequence

<220>
<223> amorce SRF-REV

<400> 4
aaattcgctt ccaggcttct g 21

<210> 5
<211> 37
<212> DNA

<213> Artificial Sequence

<220>
<223> amorce TETP1

<400> 5
gttgtatcga tgatgaaata ctgaatttta aacttag 37

<210> 6
<211> 34
<212> DNA
<213> Artificial Sequence

<220>
<223> amorce TETT1

<400> 6
tttaatggat ctagaagatt tgaattcctg ttat 34

<210> 7
<211> 26
<212> DNA
<213> Artificial Sequence

<220>
<223> amorce pC194cmfwd

<400> 7
agaaagcaga caggtaaccc tcctaa 26

<210> 8
<211> 26
<212> DNA
<213> Artificial Sequence

<220>
<223> amorce pC194cmrev

<400> 8
gcaggttagt gacattaggt aaccga 26

<210> 9
<211> 24
<212> DNA
<213> Artificial Sequence

<220>
<223> amorce SRFAA5-FWD

<400> 9
aaggaatctc gcaatcattt atcg 24

<210> 10
<211> 24
<212> DNA
<213> Artificial Sequence

<220>

<223> amorce SRFAA5REV

<400> 10
cttggtgtaa gcggaatttc tgtc 24

<210> 11
<211> 2635
<212> DNA
<213> Artificial Sequence

<220>
<223> Fragment SphI-SacI de pBG106 ayant servi à la construction de BBG116 (opéron myc sous le contrôle de PrepU-neo)

<400> 11

```
gcatgcaaaa aatggtactt tcatcacttc atgttcaaaa ttacgacaaa aaacgaaacg      60
aaaactaaag aaataatact attttcaaaa aagtacaatg aaaaatttac attttttatt     120
gtactttaat aaatatacgt taatatagtg catatatgga ttatatagcc atataattta     180
tttttctgtc attatttcac tttcttaacc tcttatttta gaactgaggg aattgttgag     240
ccgaacatct tattctttac cttgcccaaa agaaagtaca gtttatcgac ctgttggttg     300
ttccagtgtt ttttgcaggt gattccagca ttcatagttg cagaagtatt atctaagtca     360
ttgttaaacc tgtatcgtct tagccagcct tattttggcg tggtaattat aggccaatct     420
caagactagc aggacaagct tccgcaaaca atagcttcta aagtagaaac tccatatgtc     480
gcgttgctca atcaagtaaa gtttttggc gagttgcaac gtcatctgtg tccgtccctg      540
gcagttttat ctgccgcggg cttctcaccc ttgctcttgt tttgttttcc tccacctaga     600
gctattcaac tataatcaaa cgcaatcaaa tcttgaacac cctcagagac atacaaacgc     660
atcaattaaa aaaagacgtt taatcgttag gcttccatta tttgagctgc aattatgaca     720
atgatcccat atgcaggtcg actctagagc ttgggctgca ggtcgagatc agggaatgag     780
tttataaaat aaaaaaagca cctgaaaagg tgtctttttt tgatggtttt gaacttgttc     840
tttcttatct tgatacatat agaaataacg tcatttttat tttagttgct gaaaggtgcg     900
ttgaagtgtt ggtatgtatg tgttttaaag tattgaaaac ccttaaaatt ggttgcacag     960
aaaaaccccca tctgttaaag ttataagtga ctaaacaaat aactaaatag atgggggttt    1020
cttttaatat tatgtgtcct aatagtagca tttattcaga tgaaaaatca agggttttag    1080
tggacaagac aaaaagtgga aaagtgagac catgagctta tgcttaggaa gacgagttat    1140
taatagctga ataagaacgg tgctctccaa atattcttat ttagaaaagc aaatctaaaa    1200
ttatctgaaa agggaatgag aatagtgaat ggaccaataa taatgactag agaagaaaga    1260
atgaagattg ttcatgaaat taaggaacga atattggata aatatgggga tgatgttaag    1320
```

gctattggtg tttatggctc tcttggtcgt cagactgatg ggccctattc ggatattgag 1380

atgatgtgtg tcatgtcaac agaggaagca gagttcagcc atgaatggac aaccggtgag 1440

tggaaggtgg aagtgaattt tgatagcgaa gagattctac tagattatgc atctcaggtg 1500

gaatcagatt ggccgcttac acatggtcaa tttttctcta ttttgccgat ttatgattca 1560

ggtggatact tagagaaagt gtatcaaact gctaaatcgg tagaagccca aacgttccac 1620

gatgcgattt gtgcccttat cgtagaagag ctgtttgaat atgcaggcaa atggcgtaat 1680

attcgtgtgc aaggaccgac aacatttcta ccatccttga ctgtacaggt agcaatggca 1740

ggtgccatgt tgattggtct gcatcatcgc atctgttata cgacgagcgc ttcggtctta 1800

actgaagcag ttaagcaatc agatcttcct tcaggttatg accatctgtg ccagttcgta 1860

atgtctggtc aactttccga ctctgagaaa cttctggaat cgctagagaa tttctggaat 1920

gggattcagg agtggacaga acgacacgga tatatagtgg atgtgtcaaa acgcatacca 1980

ttttgaacga tgacctctaa taattgttaa tcatgttggt tacgtattta ttaacttctc 2040

ctagtattag taattatcag aattgatctg cggccgcgaa ttcaagctct agaagattgg 2100

agggagctaa tgaataatct tgccttttta tttccgggac aaggttctca gtttgttggg 2160

atgggtaaaa gtttttggaa tgattttgtg ctggcgaaga gattatttga agaggcaagc 2220

gatgccatct ccatggatgt aaaaaagttg tgctttgatg gcgatatgac tgaattgaca 2280

aggacaatga atgcacagcc tgccatttta acggttagtg ttatcgctta tcaagtatat 2340

atgcaggaaa taggaattaa accgcacttt ttggcaggtc acagcttggg cgaatattca 2400

gcgcttgtct gtgcaggtgt cctttctttt caagaagccg ttaagcttat aaggcagcga 2460

ggaatactca tgcaaaatgc agatcctgag caactgggca cgatggccgc aatcacacag 2520

gtttatatcc aaccgctaca agacctgtgt acggaaattt cgacggaaga cttcccggta 2580

ggcgtggcgt gcatgaactc ggatcaacag catgtcatct ccgggtaccg agctc 2635

<210> 12
<211> 736
<212> DNA
<213> Artificial Sequence

<220>
<223> cassette "e"pbp

<400> 12

```
aaaaaatggt actttcatca cttcatgttc aaaattacga caaaaaacga aacgaaaact     60
aaagaaataa tactattttc aaaaaagtac aatgaaaaat ttacattttt tattgtactt    120
taataaatat acgttaatat agtgcatata tggattatat agccatataa tttatttttc    180
tgtcattatt tcactttctt aacctcttat tttagaactg agggaattgt tgagccgaac    240
atcttattct ttaccttgcc caaaagaaag tacagtttat cgacctgttg gttgttccag    300
tgtttttgc aggtgattcc agcattcata gttgcagaag tattatctaa gtcattgtta    360
aacctgtatc gtcttagcca gccttatttt ggcgtggtaa ttataggcca atctcaagac    420
tagcaggaca agcttccgca aacaatagct tctaaagtag aaactccata tgtcgcgttg    480
ctcaatcaag taaagttttt tggcgagttg caacgtcatc tgtgtccgtc cctggcagtt    540
ttatctgccg cgggcttctc acccttgctc ttgttttgtt ttcctccacc tagagctatt    600
caactataat caaacgcaat caaatcttga acaccctcag agacatacaa acgcatcaat    660
taaaaaaaga cgtttaatcg ttaggcttcc attatttgag ctgcaattat gacaatgatc    720
ccatatgcag gtcgac                                                    736
```

<210> 13
<211> 1339
<212> DNA
<213> Artificial Sequence

<220>
<223> cassette PrepU-neo

<400> 13

```
gcttgggctg caggtcgaga tcagggaatg agtttataaa ataaaaaaag cacctgaaaa      60
ggtgtctttt tttgatggtt ttgaacttgt tctttcttat cttgatacat atagaaataa     120
cgtcattttt attttagttg ctgaaaggtg cgttgaagtg ttggtatgta tgtgttttaa     180
agtattgaaa acccttaaaa ttggttgcac agaaaaaccc catctgttaa agttataagt     240
gactaaacaa ataactaaat agatgggggt ttcttttaat attatgtgtc ctaatagtag     300
catttattca gatgaaaaat caagggtttt agtggacaag acaaaaagtg gaaaagtgag     360
accatgagct tatgcttagg aagacgagtt attaatagct gaataagaac ggtgctctcc     420
aaatattctt atttagaaaa gcaaatctaa aattatctga aaagggaatg agaatagtga     480
atggaccaat aataatgact agagaagaaa gaatgaagat tgttcatgaa attaaggaac     540
gaatattgga taaatatggg gatgatgtta aggctattgg tgtttatggc tctcttggtc     600
gtcagactga tgggccctat tcggatattg agatgatgtg tgtcatgtca acagaggaag     660
cagagttcag ccatgaatgg acaaccggtg agtggaaggt ggaagtgaat tttgatagcg     720
aagagattct actagattat gcatctcagg tggaatcaga ttggccgctt acacatggtc     780
aatttttctc tattttgccg atttatgatt caggtggata cttagagaaa gtgtatcaaa     840
ctgctaaatc ggtagaagcc caaacgttcc acgatgcgat ttgtgccctt atcgtagaag     900
agctgtttga atatgcaggc aaatggcgta atattcgtgt gcaaggaccg acaacatttc     960
taccatcctt gactgtacag gtagcaatgg caggtgccat gttgattggt ctgcatcatc    1020
gcatctgtta tacgacgagc gcttcggtct taactgaagc agttaagcaa tcagatcttc    1080
cttcaggtta tgaccatctg tgccagttcg taatgtctgg tcaactttcc gactctgaga    1140
aacttctgga atcgctagag aatttctgga atgggattca ggagtggaca gaacgacacg    1200
gatatatagt ggatgtgtca aaacgcatac cattttgaac gatgacctct aataattgtt    1260
aatcatgttg gttacgtatt tattaacttc tcctagtatt agtaattatc agaattgatc    1320
tgcggccgcg aattcaagc                                                 1339
```

<210> 14
<211> 536
<212> DNA
<213> Artificial Sequence

<220>
<223> cassette "e"fenF

<400> 14

```
agattggagg gagctaatga ataatcttgc ctttttattt ccgggacaag gttctcagtt      60
tgttgggatg ggtaaaagtt tttggaatga ttttgtgctg gcgaagagat tatttgaaga     120
ggcaagcgat gccatctcca tggatgtaaa aaagttgtgc tttgatggcg atatgactga     180
attgacaagg acaatgaatg cacagcctgc cattttaacg gttagtgtta tcgcttatca     240
agtatatatg caggaaatag gaattaaacc gcactttttg gcaggtcaca gcttgggcga     300
atattcagcg cttgtctgtg caggtgtcct ttcttttcaa gaagccgtta agcttataag     360
gcagcgagga atactcatgc aaaatgcaga tcctgagcaa ctgggcacga tggccgcaat     420
cacacaggtt tatatccaac cgctacaaga cctgtgtacg gaaatttcga cggaagactt     480
cccggtaggc gtggcgtgca tgaactcgga tcaacagcat gtcatctccg ggtacc         536
```